(19) Europäisches Patentamt
European Patent Office
Office européen des brevets

(11) EP 3 471 832 B1

(12) **EUROPEAN PATENT SPECIFICATION**

(45) Date of publication and mention of the grant of the patent:
**05.08.2026 Bulletin 2026/32**

(21) Application number: **17816169.1**

(22) Date of filing: **21.06.2017**

(51) International Patent Classification (IPC):
***A61Q 5/00*** (2006.01) ***A61Q 5/06*** (2006.01)
***A61Q 5/10*** (2006.01) ***A61K 8/34*** (2006.01)
***A61K 8/36*** (2006.01) ***A61K 8/73*** (2006.01)

(52) Cooperative Patent Classification (CPC):
**A45D 7/06; A61K 8/33; A61K 8/347; A61K 8/36; A61K 8/736; A61Q 5/06;** A61K 2800/94; A61K 2800/95

(86) International application number:
**PCT/US2017/038616**

(87) International publication number:
**WO 2017/223246 (28.12.2017 Gazette 2017/52)**

(54) **CHITOSAN: ALDEHYDE FILM-COATED FIBERS OR HAIRS, RELATED METHODS AND COMPOSITIONS**

CHITOSAN:MIT ALDEHYDSCHICHT BESCHICHTETE FASERN ODER HAARE, ZUGEHÖRIGE VERFAHREN UND ZUSAMMENSETZUNGEN

FIBRES OU CHEVEUX REVÊTUS DE FILM DE CHITOSANE:ALDÉHYDE, PROCÉDÉS ET COMPOSITIONS ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.06.2016 US 201662352880 P**

(43) Date of publication of application:
**24.04.2019 Bulletin 2019/17**

(73) Proprietor: **Henkel AG & Co. KGaA**
**40589 Düsseldorf (DE)**

(72) Inventors:
- **SAVAIDES, Andrew**
  **Norwalk, CT 06854 (US)**
- **TASKER, Rushi**
  **Trumbull, CT 06611 (US)**
- **VAIDYA, Mona**
  **Little Ferry, NJ 07643 (US)**
- **MARTICH, Jasmine**
  **Norwalk, CT 06851 (US)**

(56) References cited:
**WO-A1-2013/165424**
**WO-A1-2014/152455**
**WO-A2-2012/175221**
**DE-A1- 10 259 199**
**JP-A- 2007 262 001**
**US-A1- 2009 226 381**
**US-A1- 2011 165 109**
**US-A1- 2012 031 421**
**US-A9- 2016 151 271**
**US-B2- 7 597 898**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 471 832 B1

## Description

### BACKGROUND OF THE INVENTION

Chitosan is a derivative of chitin, a compound usually isolated from the shells of crustaceans such as crab, lobster and shrimp. Chitosan is the soluble form in aqueous acidic solutions or partially the deacetylated derivative of chitin, *i.e.,* it consists of α (1 ~4)-linked 2-acetamido-2-deoxy-β-D-glucopyranose and 2-amino-2-deoxy-~D-glucopyranose residues. Chitin and chitosan are abundant, renewable and sustainable polymers and have excellent properties such as, biodegradability, bio-compatibility, non-toxicity, and adsorption to hair, skin and nails. Most chitosans are derived from crustaceans shells; they therefore can be marketed as "natural" to consumer products end users.

In addition, chitosan is well known for its interesting and varied chemical and biological properties which have led to an expanding number of industrial, medical and personal care applications. In various forms, chitosan has been used in hair care products to smooth, condition or "fix" the hair by forming a film on the hair.

Conventional chitosan films are prepared by delivering chitosan in a liquid or semi-liquid carrier to the hair, and allowing the liquid to volatilize, leaving behind a layer of chitosan. Such deposits have a transient effect on hair or fibers. WO 2013/165424 A1 discloses a method for holding a keratin fiber in a desired shape or style, comprising: applying a sol-gel composition to a keratin fiber; said sol-gel composition comprising an aqueous dispersion of (a) epoxy- functionalized inorganic oxide particles; (b) a cosmetic particulate selected from the group consisting of inorganic particulates, organic polymeric particulates, pigments, lakes, silicon-based particulates, and combinations thereof; and (c) chitosan or a chitosan derivative; imparting a desired shape or sty le to said keratin fiber; and allowing said sol-gel composition to dry.DE 102 59 199 A1 teaches a process where the hair agent is polymerized directly on the hair or fiber. However, DE 102 59 199 A1 teaches the use of a bi-copper, oxygen-dependent, metalloenzyme to catalyze this polymerization

However, there remains a need in the art to develop a method of treating hair using an improved chitosan-containing composition that is effective and long lasting, but which can be presented to the consumer as safer and "natural".

### BRIEF SUMMARY OF THE INVENTION

The invention includes methods of forming a semi-permanent film on a hair fiber comprising topically applying an effective amount of a composition to the hair and exposing the hair to a temperature of 60°C to 190°C thereby forming a film on the hair, wherein the composition comprises a chitosan, an aldehyde-bearing compound, and an organic acid and wherein the aldehyde-bearing compound is selected from a phenolic aldehyde.

Also included are the compositions that form the film, specifically compositions capable of forming a semi-permanent film in situ on a fiber in the presence of heat comprising a chitosan, an aldehyde-bearing compound, and an organic acid, wherein the aldehyde-bearing compound is selected from a phenolic aldehyde.

Other, related methods are within the scope of the invention. For example, methods of semi-permanently altering the structure of a hair; of providing a semi-permanent UV protective film to a fiber; of semi-permanently increasing the tensile strength of hair; and of semi-permanently reducing color leaching from a color treated hair.

Included within the scope of the invention are chitosan:aldehyde Schiff base polymer films on a fiber prepared by combining a chitosan and an aldehyde-bearing compound in the presence of water and an organic acid on a hair surface and exposing the hair surface to a temperature of 60°C to 190°C thereby forming a film on the hair surface.

### BRIEF DESCRIPTION OF THE SEVERAL VIEWS OF THE DRAWINGS

The foregoing summary, as well as the following detailed description of preferred embodiments of the invention, will be better understood when read in conjunction with the appended drawings. For the purpose of illustrating the invention, there is shown in the drawings embodiments which are presently preferred. It should be understood, however, that the invention is not limited to the precise arrangements and instrumentalities shown. In the drawings:

Fig. 1 is a DSC thermogram of chitosan, trade name HYDAMER™ HCMF (minimum 80% deacetylated; MW 50,000 to 1,000,000 g/mol), identified as "1" in Example 2.
Fig. 2 is a DSC thermogram of chitosan, trade name HYDAMER™ CMFP (minimum 805 deacetylated; MW 500,000 to 5,000,000 g/mol), identified as "2" in Example 2.
Fig. 3 is a DSC thermogram of chitosan, trade name HYDAMER™ HCMF-acetate film, identified as "3" in Example 2.
Fig. 4 is a DSC thermogram of chitosan, trade name HDAMER HCMF-vanillin-acetate film, identified as "4" in Example 2;
Fig. 5 is a DSC thermogram of Vanillin from Example 2;
Fig. 6 is an FTIR spectra of chitosan (HYDAMER™ HCMF);

Fig. 7 is an FT-IR spectra of vanillin;
Fig. 8 is an FT-IR spectra of HYDAMER™ HCMF-Acetate Film; and
Fig. 9 an FT-IR spectra of HYDAMER™ HCMF Vanillin - Acetate Film.

## DETAILED DESCRIPTION OF THE INVENTION

The invention described herein includes methods and compositions that, when practiced, develop a chitosan:aldehyde Schiff base polymer film on a fiber, especially a hair. The film provided by the disclosed methods and compositions is capable of supplying certain enhanced benefits to human hair and/or other types of fibers, including smoothness, increased volume, shine, and luster, protection from the sun or pollution, preservation/protection of color applied to hair, alteration of the structure of the hair, *etc.*

The film, methods and composition of the invention are useful in the hair, skin or nail care area, as well as the activities related to the treatment, processing and/or preservation or conservation of textile fibers, especially natural textile fibers, such as wool, leather, linen, fur, silk, flax, etc.

The invention includes methods of forming a film on a fiber. The fiber may be a plant- or animal- derived fiber or a synthetic fiber. For example the fiber may be mammalian hair, a human hair, feather, cotton, wool, leather, fur, cashmere, whiskers, baleen, linen, flax, silk, a synthetic polymer fiber, bamboo fiber, and combinations of the same. Fibers of any diameter may be employed in the method of the invention. Herein "fiber" and "hair" are used interchangeable.

The film formed may be continuous on the fiber surface or discontinuous, and may be of any thickness.

In the practice of the method, a composition is topically applied to the fiber. The composition may be applied by any means in the art, including, for example, combing, wiping, smudging, painting, smearing, blotting, soaking, and spraying.

The film-forming composition for use in the methods of the invention includes chitosan. Any chitosan may be used. In an embodiment, it may be preferred that chitosan having a deacetylation of about 50% to about 100%, about 60% to about 80% and about 70% to about 95% is used, and molecular weights of about 50,000 to 1,000,000 g/mole, 300,000 to 2,000,000 g/mole and 500,000 to 5,000,000 g/mole.

The film forming composition further includes an aldehyde-bearing compound (ABC). By ABC it is meant any compound having one or more aldehyde functional groups that are available to react in situ on the fiber with the nitrogen atoms of chitosan to form a Schiff base imine. In some embodiments, it may be preferred that the ABC is vanillin, p- vanillin, o-vanillin, or ethylvanillin. Other suitable ABCs may include, for example without limitation, benzaldehyde, formaldehyde, acetaldehyde, (R)-carvone, cinnamaldehyde, glyoxylic acid, 3-methyl-3 phenyl glycidic acid ethyl ester, acetaldehyde, capraldehyde, piperonal citral, undecanal, octanal, heptanal, nonanol, dodecanal, ethyl 3-(4-methylphenyl)oxirane-2-carboxylate, unadecalactone, oleic aldehyde, glyoxal, glutaraldehyde, and methyl nonyl acetic aldehyde. Combinations of different ABCs may also be used in the composition.

The film forming composition also includes an organic acid such as a mono- or dibasic acid. Any may be suitable. In some embodiments, the preferred organic acid is acetic acid. Other organic acids that may be used include, without limitation, formic acid, glycolic acid, lactic acid, ascorbic acid, pyruvic acid, salicylic acid, citric acid, adipic acid, succinic acid, malic acid, tartaric acid, fumaric acid, glyoxilic acid and combinations thereof.

In some embodiments of the invention, the composition may include a component that provides a specialized attribute to the final film, for example, UV filters for sunscreen or to increase color protection, light reflectants or dispersants to enhance shine, pigments or colorants to impart color to the fiber or hair.

In the preparation of the composition, it may be preferred that the ratio of chitosan to ABC is about 4:1 (w/w %) although other ratios can be used and may vary depending on the specific types of chitosan, acid and ABC used and their specific reactivities. Table A shows exemplary ranges of the chitosan, ABC and an organic acid that may be suitable for use in the composition.

Table A

| Components | Amount (w/w %) |
|---|---|
| Chitosan | about 0.05 to about 5 |
| | about 1.5 to about 4 |
| ABC | 0.013 to about 5 |
| | about 0.5 to about 3 |
| Organic acid | about 0.5 to about 3 |
| | 0.9 to about 2 |

Other additives may be included in the composition, such as those ordinarily found in personal care and/or textile care formulations. Such additives may also include sensates, fragrances, colorants, metal chelators, pigments, water, oil, waxes, alcohols, surfactants, UV filters, light reflectants or dispersants, glitter, conditioners, and preservatives.

In embodiments of the invention, it may be preferred that the final pH of the composition is about 3.0 to 5.0 or about 3.5 to about 4.5. To arrive at this pH, various alkali compounds may be added to adjust the composition as is known in the art. Such alkali compounds may include ammonia, sodium hydroxide, potassium hydroxide, monoethanolamine, diethanolamine, diisoproanolamine, triethanolamine, and isopropanolamine.

The composition may be prepared in any dispensed product format desired or known in the art. For example, it may take the form of a liquid, semi-liquid, gel, spray, mist, foam, mousse, paste, crème, emulsion and the like. In some embodiments, the composition is prepared as a loose or compressed powder, granules, pellets, etc., to which the end consumer or the distributor may add a liquid or semi-liquid solvent or carrier. Such forms may be desirable to reduce shipping costs (less weight to transport).

In the practice of an embodiment of the invention, a composition containing any of the components described above is prepared and applied topically ("to the surface of") the fiber. Heat is applied to the fiber - preferably at a temperature of about 50°C or greater. Alternatively, the temperature is about 60°C to about 190°C or about 100°C to about 160°C.

The heat may be applied by any means known or developed in the art. It may be applied directly or ambiently. Suitable devices for providing heat include, without limitation, a hair dryer, a curling iron, a flat iron, a curling rod, a pre-heated hot roller, a blower, an oven, a heated plate, and combinations thereof.

In general, it may be preferred that the fiber is exposed to the heat for duration of time ("drying time") sufficient to drive off at least portion of the aqueous solvent from the fiber. In some embodiment, this may mean that the fiber is about 60% to about 100% dry, about 70% to about 95% dry, and/or about 80% to about 90% dry. The drying times to achieve these percent drynesses will vary. As an example, in the case of a head of shoulder length hair of average thickness, the drying time may be about 5 minutes, about 10 minutes, or about 20 minutes to achieve these results.

Upon removal of at least a portion of the aqueous solvent, a chitosan:aldehydes Schiff base polymer film will be formed on the fiber. Without binding themselves to a theory, the inventors propose that the film is formed by the following exemplary reaction scheme, wherein the chitosan is a low molecular weight chitosan of 80% deacetylation, the organic acid is acetic, and the ABC is generically represented as RCHO :

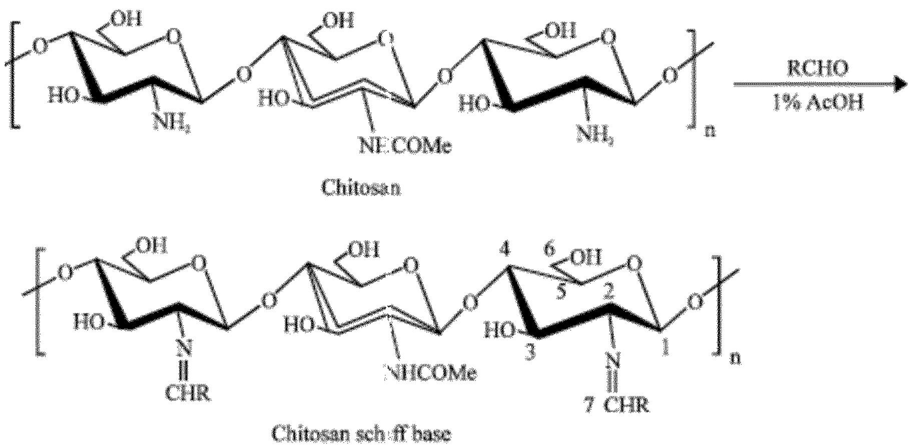


Scheme 1

The chitosan: aldehyde Schiff base polymer thereby formed is likely to be highly adherent to fibers, especially natural fibers like skin and hair, because of its ability to react with the side peptide terminals present in the amino acids present on the hair cuticle (lysine, aspartic acid, glutamic acid, proline and cysteine). Reaction Scheme 2 is an example of the *in situ* development of the chitosan: aldehyde Schiff base polymer when the chitosan is a low molecular weight and 80% deacetylation and the ABC is vanillin:

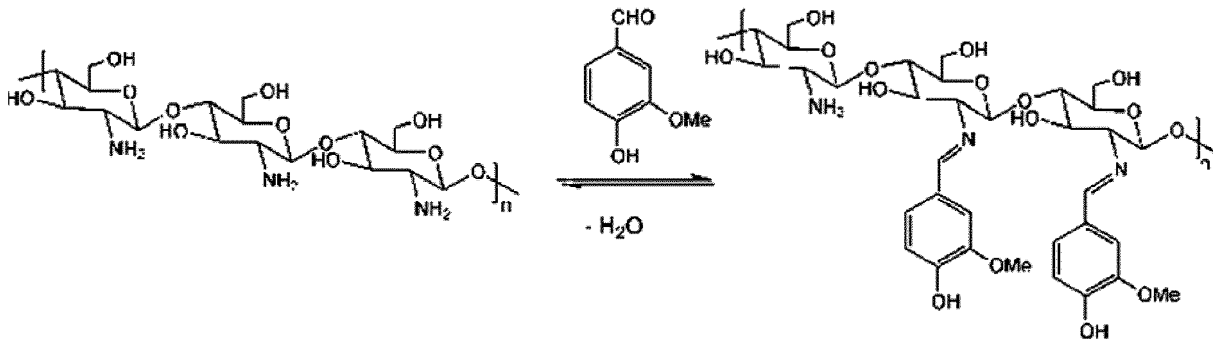

C V $C_nV$, n=0.5, 1.5 1-5

Scheme 2

As a consequence of the high adhesively of the chitosan: aldehyde Schiff base polymer film of the invention to fiber, especially mammalian hair, the films formed and the effects provided by the films are, in an embodiment, semi-permanent. By semi-permanent it is meant that the film is present on the hair or fiber and/or the effect provided by the film to the fiber or hair ("film effects") is observable after the hair is washed in a conventional manner at least 3 times or more. For example, the film or the film effects may be observable for about 3-about 20 washings, for about 5-about 15 washings, for about 8-about 12 washings. The degree of permanency (*e.g.,* 3 washings versus 20 or more) will vary depending on the specific hair type and the end effect for which the film is applied.

The methods of the invention using the composition in any and/or all of the variations described above to form a film may be used to achieve any number of end results or combinations of end results. The methods may be used to semi-permanently alter the structure of the hair. By "alter the structure" it is meant to modify one or more observable aspect of the hair, such as to make it less frizzy, less fine, to reduce "fly aways", to increase shine and luster, to increase softness, to increase resistance to humidity, etc.

The methods may be used to semi-permanently deliver to the hair or fiber a specialized compound that imparts an advantage - for example a UV filter, thereby reducing and/or eliminating UV damage to the hair or fiber. Other specialized compounds may be, for example, fragrances, mico-glitter, or light reflectants, light diffusers, and the like.

Also contemplated are methods of increasing the tensile strength of hair or fiber, resulting in, for example, less breakage when it is mechanically manipulated, such as when brushed or during a weaving or knitting process.

The methods may be used to provide color protection (that is, reduce or prevent leaching of color from hair that has been previously dyed, thereby increasing the time between hair coloring or recoloring sessions). The reduction in color leaching or loss is observable and is quantifiable using the color metric set forth by the International Commission on Illumination. Under such metric, hair or fiber coated with the film of the invention may have, for example, a Delta-E of about 2.0 or less after three washings.

In addition, the benefits and advantages provided to the hair or fiber by the above-described compositions, methods and chitosan:aldehyde Schiff base polymer films may be further enhanced, modified, refined and/or otherwise altered by coupling the use of the composition that forms the chitosan:aldehyde Schiff base polymer films on hair with an anionic formulation that is applied to hair before or after the film-forming composition or that is alternatively applied to the hair or fiber at the same time or very close in time. Such benefits may be over and above the already enhanced benefits provided by the chitosan:aldehyde Schiff base polymer films on hair and may include, for example, wet and dry conditioning, smoothness, cuticle/hair shaft repair, minimization of porosity of hair cuticle, improved sheen and silky feel of hair, thermal and mechanical protection, moisturization, volume, body and static electricity. The anionic formulation as such is not covered by the claims

The anionic formulation may include at least one polyanionic material and a carrier. Anionic materials may be water soluble anionic materials, such as natural anionic polysaccharides, synthetic anionic polymers, synthetic anionic copolymers, anionic proteins, oxidized proteins, anionic silicones, anionic surfactants (*e.g.,* those having $SO_3$-, $SO_4$ - and/or COO - functional groups in their structures) or amphoteric surfactants. The carrier may include a foam, liquid or gel that includes or wholly contains water, alcohols, water-alcoholic mixtures, waxes, oils, *etc.*

In some embodiments, the carrier may be a "dry" carrier, such as a powder, talc, minerals, cornstarch, carbon granules, which can either be applied to hair in its dry form, or reconstituted by the user with water, alcohol, *etc.,* before application.

Specific polyanionic materials may include polyacrylic acid, polystyrene sulfonate, VA/crotanates copolymers, acrylates copolymers, polyaspartate, sodium cetearyl sulfate, sodium lauroyl glutamate, glyceryl stearate, acrylamide/-sodium acryloyldimethyltaurate/acrylic acid copolymer, acrylates/methoxy PEG-10 maleate/styrene copolymer, sodium acrylates/beheneth-25 methacrylate crosspolymer (and) hydrogenated polydecene (and) lauryl glucoside, dimethiconol

(and) TEA-dodecylbenzenesulfonate, acrylates/steareth-20 methacrylate copolymer, dehydroxanthan gum, dimethicone PEG-7 phosphate, alkyl ether sulfates, sulfonic acids, isethionates, sulfosuccinates and sulfosuccinamates, alkyl sulfates, glyceryl esters/derivatives, sarcosinates and sarcosine derivatives, sodium laureth sulfate, sodium $C_{14-16}$ olefin sulfonate, sodium laureth sulfate, sodium lauryl sulfate, ammonium laureth sulfate, sodium lauroyl glycinate, sodium methyl cocoyl taurate, dodecylbenzene sulfonic acid (and) water, glyceryl stearate citrate, disodium cocoyl glutamate, TEA-cocoyl alaninate, sodium $C_{14-16}$ olefin sulfonate, dimethiconol (and) TEA-dodecylbenzenesulfonate, sodium methyl cocoyl taurate, sodium coco-sulfate, sodium cocoyl isethionate, sodium coco-sulfate, sodium methyl lauroyl taurate, disodium cocoamphodiacetate, TEA-cocoyl glutamate, dimethiconol (and) TEA-dodecylbenzenesulfonate (XIAMETER® MEM-1788 Emulsion), dimethiconol (BELSIL® DM 3112 VP), dimethicone PEG-7 Phosphate (PECOSIL® PS-112), natural gum arabic, xanthan gum, biossacharide gum-4, carrageenan (I, k and $\lambda$), alginic acid, pectin, lactobionate, hyaluronic acid, heparin, soybean-soluble polyssacharide, hydroxypropyl guar, carboxymethyl hydroxypropyl guar, carboxymethyl guar, 18-MEA, cholesterol sulfate, Keratec IFP-HMW, Keratec NKS, Keratec PEP, Keratec IFP, oxidized keratin, cocoyl hydrolyzed collagen and mixtures thereof.

In an embodiment, a benefit provided by the formation of the chitosan:aldehyde Schiff base polymer films on a fiber includes increasing or enhancing the fiber's receptivity and ability to retain color dye over time, especially acids dyes, such as Acid Black, Acid Blue, Acid Green, Acid Red, Acid Violet, Acid Yellow, and Acid Orange dyes. The acid dyes are water soluble or in ethanol and are highly anionic due to their sulfonic acid groups. Other dyes that may be used include acid leveling dyes, which are small in size, diffuse quickly into hair and provide uniform color. The color dye may be for a textile fiber or for a hair.

As an example, relating to hair coloring, semi-permanent hair coloring is performed by first forming the chitosan:aldehyde Schiff base polymer films on the hair using any of the methods or composition described herein. Subsequently, one applies to the hair an aqueous or aqueous/alcoholic solution of the dye(s) at pH less <8 (potentially more preferred at pH 4-6). The dye solution remains on the hair for about 2 to about 5 minutes, or about 15 to about 20 minutes, depending on the condition of hair. The hair is then rinsed with water.

The adsorption of these dyes onto chitosan treated hair is strong due to the high cationic charge of hair provided by the chitosan:aldehyde Schiff base polymer films. It is believed that the ionic interactions of the sulfonic groups -($SO_3$) of the acid dyes and the -$NH_3^+$ of the chitosan:aldehyde schiff base are strong, resulting in long lasting uniform semi-permanent hair color lasting over several shampoos compared to colored hair without the polymer film.

Typically, semi-permanent color of hair is acceptable at pH > 8 on hair processed for 20-40 minutes following with rinsing. There is conceivable damage to the fiber due to the high pH and longer processing times.

In a method of the invention that includes treatment with the anionic formulation, supra, the anionic formulation is prepared and applied to the hair or fiber that one wishes to treat. The hair or fiber may be dry, damp or wet. For better results, the application is carried out in a manner that provides even and uniform distribution of the anionic formulation. Suitable mechanisms of application can include spraying, pouring, wiping, combing with implement or fingers, brushing, or any combination of these.

In an embodiment of the invention, the hair to which the anionic material has been applied is left to process for a minute or more (*e.g.,* 1 to 10 minutes or 5 to 15 minutes). Subsequently, any of the chitosan and aldehyde-bearing compound compositions described above is applied to the hair. Heat is applied to the hair or fiber as described in the methods above, and a complex coacervate that includes the chitosan: aldehyde-bearing polymer film and the anionic material is formed.

In various embodiments of the invention the complex coacervate may be formed by applying the chitosan: aldehyde-bearing polymer film forming composition to the fiber before application of the anionic material in a two-step process (preferably followed by heat application), or it may be formed by co-application of the anionic material and the film forming composition, for example, using dispenser with two nozzles.

Also included in the inventor are various hair care assemblies and kits to treat, dyes or otherwise deliver or deposit enhancements or additives to hair. Such kits may include "communication devices" that provide information related to the kit, such as instructions. These devices may take any form, such as a writing, video, audio, etc.

The kits and assemblies may also include accessory useful in the use or application of the contents of the kit or hair care generally. Such accessories can include gloves, a hair clip, a comb, a plastic cap or hat, a timer, a swab, a brush, and a mirror.

## EXAMPLES

### Example 1 - Preparation and Evaluation of Films

The aqueous compositions 1, 2, A, B, C, D, E, and F shown in Table 1 containing different ratios of Chitosan: Aldehyde-Bearing Compound with 1% acetic acid were prepared so they could be evaluated for the quality and characteristics of the film and its effect on hair. The aldehyde-bearing compound used was vanillin and the chitosan

had a molecular weight of 50,000-1,000,000 g/mole with 95% deacetylation.

The chitosan is weighed and added slowly into a 1% acetic acid solution. The mixture is stirred until the chitosan goes into solution. The vanillin is added and mixed further until all vanillin dissolves.

Table 1

| Composition | Chitosan (wt%) | Vanillin (wt%) | Acetic Acid (wt%) |
|---|---|---|---|
| 1 | 0.50 | 0 | 1 |
| 2 | 0 | 0.50 | 1 |
| A | 0.45 | 0.05 | 1 |
| B | 0.40 | 0.10 | 1 |
| C | 0.35 | 0.15 | 1 |
| D | 0.30 | 0.20 | 1 |
| E | 0.25 | 0.25 | 1 |
| F | 0.20 | 0.30 | 1 |

Fresh aqueous solutions of the different ratios of total 0.5% w/w Chitosan: Vanillin composition mixtures shown in Table 1 were placed in multi-well glass plates and glass Petri dishes in an oven for 2-3 hours @ 110-112°C. The films were cooled down at room temperature and were visually examined. The films were stored in small capped vials and were analyzed subsequently.

The film characteristics analyzed were: flex/bending strength, thickness, shine/luster, and water solubility behavior. The films were further analyzed by (DSC) Differential Scanning Calorimetry, and (FT-IR) Fourier Transfer Infrared.

Overall, all composition mixtures yielded good, luminous, shiny films except with composition 2 where vanillin recrystallized out. All films were sparingly soluble in water. The films from the compositions A, B, C, and D films were found to have the best flex/bending strength. The film from composition 1 was weak and had a poor flex/bending strength; it was the most brittle compared to the other films.

The thickness of each film was measured with the Mitutoyo Caliper (Dial Thickness Gauge). No differences in film thickness were noted. The measured thickness ranged between 0.035-0.04 mm.

Example 2 - Evaluation of films by Differential Scanning Calorimetry (DSC)

The chitosan films were prepared as described above by placing fresh solutions described into Petri dishes in the oven at 110-112°C until all the liquid has evaporated. The films were cooled down at ambient temperature, dried further, cut into small pieces and capped into small glass vials. Samples of 6-8 mg were weighed into aluminum pans, sealed and analyzed with a Perkin Elmer Diamond DSC, heat from 50C° to 475C° at 100C°/min. The results are tabulated in Table 2 below.

Table 2

| | $T_1$ (C°) | $\Delta H_1$ (J/g) | $T_2$(C°) | $\Delta H_2$ (J/g) |
|---|---|---|---|---|
| HYDAMER™[1] HCMF* | 214.24 | 124.77 | 369.88 | -325.76 |
| HYDAMER™ CMFP** | 214.28 | 161.25 | 378.18 | -149.59 |
| HYDAMER™ HCMF -acetate film*** | 187.37 | 494.40 | 347.698 | 26.05 |
| HYDAMER™ HCMF-vanillin-acetate **** | 170.79 | 103.76 | 318.03 | 137.61 |

[1] HYDAMER™ is a trade name of a chitosan product line available from Chitinor AS, Haugesund, Norway.
* low molecular weight chitosan (50,000 to 1,000,000 g/mole); minimum 80% deacetylation.
** high molecular weight chitosan (500,000 to 5,000,000g/mole); minimum 80% deacetylation.
*** films prepared in presence of 1% (wt/wt) acetic acid.
***** chitosan: vanillin 4:1.

The DSC thermograms depict that the chitosan is an amorphous and crystalline polymer. The DSC thermograms

show two major transitions for both low and high molecular weight chitosan polymers. The first transition is endothermic with a melting $T_m$ ($T_1$) around 214C° depicting the amorphous region of the polymer and the second transition is exothermic with a crystallization $T_c$ ($T_2$ ) around 369 - 379 C°. Based on the heat of denaturation $\Delta H_1$ and heat of crystallization $\Delta H_2$ of the high and low molecular weight polymers the low molecular weight chitosan is more crystalline than the high molecular weight. Whereas the high molecular weight chitosan has an equal amorphous and crystalline regions.

Referring to Figure 1, the DSC thermograms show that the Chitosan-Acetate film formed with the addition of 1% acetic acid have affected the high crystalline region of the low molecular weight chitosan showing a shift in the crystallization temperature $T_2$ from 369.88 C° to 347.98 C° and decrease in the heat release of $\Delta H_2$ from -325.76 to - 26.05 J/g. It appears also that the amorphous region of the polymer has been modified and increased. The melting Tm ($T_1$) temperature has decreased from 214.24 C° to 187.37 C° and an increase in denaturation heat $\Delta H_1$ to 494.40 from 124.77 J/g. The Chitosan-Vanillin-Acetate film shows that the chitosan polymer has been changed to a new higher molecular weight with equal levels of amorphous and crystalline regions. It appears that the Schiff base formation produced a much more orderly structure than the acetate having a balanced amorphous and crystalline region similar to the high molecular chitosan. The endothermic transition $T_m$ ($T_1$) shifted to 170.79 C°, lower than the Chitosan-Acetate $T_m$ ($T_1$) of 187.37 C° depicting further changes to the Chitosan-Acetate polymer structure. The denaturation heat ΔH1 for the Chitosan-Vanillin-Acetate film is 103.76 J/g this is compared to 124.77 J/g for the Chitosan Polymer. The crystallization temperature $T_2$ has shifted to 318.02 C° from 347.98 C° with a much greater $\Delta H_2$ heat release of -137.61 J/g. The incorporation of vanillin into the Chitosan-Acetate produced a much more uniform amorphous and crystalline polymer. The DSC thermograms show that the composition C films do not have free vanillin which has a transition of Tm 84.62 C°. Therefore the Chitosan-Vanillin-Acetate films have surprisingly unique physical and chemical characteristics on hair.

Example 3- Evaluation of Films By FT-IR

Referring to Figures 2 to 9, the FT-IR spectra of chitosan shows the O-H stretch at about 3400 cm-1 is shifted to 3350 cm-1 in the modified chitosan -vanillin acetate film Figure 9 depicting intermolecular hydrogen bonding. Also the N-H stretch is shifted from 3450 cm-1 to 3350 cm-1 indicating secondary amide. The FTIR spectra of the Chitosan Vanillin Acetate film shows a C-H stretch from the methylene groups. The spectra shows also the imide HC=N stretch at 1645 cm-1 and N-H bending of secondary amides at 1592 cm-1 . Also C=C stretching from the aryl ring is shown at 1514 cm-1. The spectra show C-O-C stretching at 1283.47 cm-1. Both Figures 2 and 4 of chitosan and chitosan acetate do not show any, C=O, C=C stretching and N-H bending of secondary amides confirming the modification of chitosan structure with vanillin.

Example 4 - Evaluation of Compositions of the Invention on Hair

Straight slight wavy normal and 20 volume color treated hair swatches were shampooed with a cleansing shampoo twice, blotted to remove excess water. Each of the composition mixtures were applied on the hair and processed for 20 minutes at ambient temperature. The hair was towel blotted and dried under a hair dryer to about 95%.

The hair was crimped afterwards with a preheated crimper at 144C° (290F°) for 15-20 seconds for each section. The crimped hair samples were evaluated immediately and placed them in a 90% relative humidity at 24.4C° for 24 hours and evaluated again. All hair swatches before placing them in the humidity chamber were shiny, lustrous and had a nice wave pattern impression of the crimper. No visible flaking or powder was visually detected on hair. The hair swatches with the highest shine order were A>2>B>C>1. The level of vanillin: chitosan as it increased from (10:90)% to about (40:60)% of the total 0.5% (w/w) improved the appearance of hair dramatically. The swatches with the mixture A of Vanillin:Chitosan of (20:80)% or 1:4 ratio had the highest shine, and luster. All swatches after 24 hours in the humidity still had smooth and wavy hair except with the control that had all lost all wave. The swatches were further washed with a cleansing shampoo for a total of seven shampoos. The hair swatches were evaluated after each shampoo. The swatches 2, A, B and C had better wave and conditioning for about 5-7 shampoos. The wet combing of hair is detected on hair even after seven shampoos. The chitosan treated hair swatches had effect for about 2 shampoos.

Example 5 - Preparation and Use of Composition for Normal Frizzy Hair and for Chemically Damaged Hair

A composition of the invention is prepared by combining the ingredients in the amount listed in Table 3:

Table 3

| | w/w% | w/w% (range) |
|---|---|---|
| Chitosan | 0.40 | 0.01-4.0 |
| Vanillin | 0.10 | 0.04-1.0 |

(continued)

| | w/w% | w/w% (range) |
|---|---|---|
| Acetic acid | 1 | 0.50-10 |
| Phenoxyethanol | 1 | 0.50-2.0 |
| Fragrances | 0.1 | 0.05-0.25 |
| PPG-26-Buteth-26 (and) PEG-40 Hydrogenated | 0.20 | 0.10-0.50 |
| Castor Oil | | |
| Water QS | 100 | 100 |
| pH QS with 20 % NaOH | 3.5 | 3.0-6.5 |

The composition of Table 3 is a leave-in treatment for normal frizzy or colored hair. The composition is applied thoroughly onto shampooed and towel-dried normal or colored curly frizzy hair. The hair is combed to ensure even application and the treatment was left on for 5 minutes, more preferably about 15-20 minutes.

The hair is dried and styled with a blow drier to about 95% dryness with high heat setting using a brush. For optimum smoothing results the hair is flat ironed at low heat setting of about 280- 300F°. The hair has a natural healthy soft clean feeling where synthetic polymers may leave a perceptible coating that may flake. The hair has more shine, sheen, luster, less frizz, volume and healthy movement. The hair also has excellent wet conditioning properties depicted by the ease of wet and dry combing. The hair is shampooed after 48 hours and the curls are more open and more defined. The effects last several shampoos up to about 5-7.

Example 6- Alternative Use of Composition for Normal Frizzy Hair and for Chemically Damaged Hair

The composition of example 5 was applied thoroughly onto shampooed and towel-dried hair. The hair is combed to ensure even application and left on for 5 minutes, more preferably about 15-20 minutes. The hair is dried and styled with a blow drier to about 100% dryness with high heat setting using a brush. The hair is shampooed afterwards and styled. The hair has more, shine, sheen, luster, less frizz, volume and healthy movement. The hair also has excellent wet conditioning properties depicted by the ease of wet and dry combing. The effects lasted a few shampoos of 2-3.

Example 7- Treatment of Permed/Waved or Straightened Hair

The composition of example 5 is applied on permed and thermally straightened hair with ammonium thioglycolate after neutralization. The permed hair was dried with a diffuser and the straightened hair was dried with a blow drier to about 90% dryness and then was ironed at low heat setting of about 137.78-148.89°C (280-300°F). The hair was shampooed after 48 hours. Initially and after 48 hours the permed hair has more shine, luster, less frizz and more defined curls that lasted after several shampoos. The thermally straightened hair has better dry and wet conditioning, more fiber alignment, less frizz, less volume, more shine, luster and more straighter. These effects lasted for at least a month or 12-18 shampoos.

Example 8- Use of Composition For Styling/Fixing Hair

The composition of Example 5 is applied on shampooed and towel-dried normal, chemically straight, wavy or curly hair for 5 minutes more preferably about 15-20 minutes. The hair is dried with a blow dryer at medium setting and is styled with a curling iron, crimper or preheated rollers or a diffuser. The hair has a nice hold set and good instant waves or more defined waves that showed good humidity resistance and stability versus a water set. Control water set hair swatches in 90% relative humidity had lost almost 95% of their style after 48 hours. The treated swatches had maintained at least 80% of their style after 48 hours in the 90% relative humidity.

Example 9 - Use of the composition for Hair Color Protection

Normal hair was bleached followed with semi-permanent color dying containing Basic Dyes Yellow 87, 57, Red 76, Violet and HC blue for 20 minutes. The dyed hair was rinsed, and shampooed twice, rinsed and towel-dried.

The composition of Example 5 is applied thoroughly onto shampooed and towel-dried colored hair. The hair is combed to ensure even application and left on for 5 minutes, more preferably about 15-20 minutes. The hair is dried and styled with a blow drier to about 95% with high heat setting using a brush. For optimum smoothing results the hair is flat ironed at low heat setting of about 137.78-148.89°C (280-300°F).

The hair has initially a natural healthy soft clean feeling and more vibrant color than the control. The hair has more shine, sheen, luster, less frizz, volume, deeper color and healthy movement. The hair also has excellent wet conditioning depicted by the ease of wet combing. The treated hair had more color retention than the untreated after multiple washes. The color protection for single treatment has a color protection up to 5-7 shampoos. The color protection is more effective if the hair is treated in between shampoos without ironing.

The hair color protection is shown on Table 4 below from a single treatment on semi-permanent colored hair. The hair swatches were dried and the Hunter Color Coordinates (L, a, b) were determined before and after shampooing for the untreated and treated hair swatches. The ΔE was calculated after each shampoo for the treated and untreated swatches shown below. Based on the ΔE values the single treatment provided more color protection than the untreated hair swatches from washings.

Table 4

| HAIR SAMPLES | ΔE |
|---|---|
| Untreated Semi-permanent Dyed* Hair + 3x Shampoos | 3.22 |
| Treated Semi-permanent Dyed* Hair + 1x Shampoos | 1.08 |
| Treated Semi-permanent Dyed* Hair + 3x Shampoos | 2.31 |
| *Vero Pak Color Intensity Ruby Red | |

The color protection on permanent dyed hair treated with the composition of example 5 has also been observed on swatches. The color leaching is less with the treated swatches than the untreated. Again, the color protection is maximized when the hair is treated in between shampoos.

Example 10 - Pre-Treatment of Hair Prior to Treatment With Composition of the Invention (The Pre-Treatment in itself is not covered by the claims)

The treatment effects are enhanced when the hair is pre-treated with 0.5% $NH_4OH$ or other alkali. The alkali solution is applied onto the hair for 5 minutes and then is rinsed and towel blotted. Then the hair is treated following Examples 4-8. This pre-treatment is especially for very frizzy and unruly resistant hair.

For extremely damaged hair including singly or multi bleached hair the addition of divalent metal ion increases the performance of the treatment. The ratio of Chitosan: Divalent ion is 8:1. The divalent metals Zn, Mg, Mn, and salts of acetate, citrate, nitrate, sulfate are used at 0.05 %. Excellent results have been observed on singly and multi bleached hair using the composition of example 11 (below) using the treatment methods of examples 4-8. The hair has more visible strength, shine, luster and movement as well as better smoothness.

Example 11 - Protection of Semi-Permeant Color Applied to Hair

Blonde hair tresses were obtained and bleached two times with 1:2 Verolight Lightening Powder with 40 Vol. Veroxide developer for 40 minutes covered in aluminum foil. They were then rinsed for 2 minutes with lukewarm water and towel blotted dry.

The bleached hair tresses were colored with semi-permanent Joico Intensity Peacock Green two times, so that the color deposition was even. The tresses were dried to about 100 % with a dryer, and the hunter color coordinates L, a, b were measured.

Half of the colored tresses were then treated with the composition of Table 5. Half were left untreated.

Table 5

| | w/w% | w/w% (range) |
|---|---|---|
| Chitosan | 0.60 | 0.01-4.0 |
| Vanillin | 0.15 | 0.04-1.0 |
| Acetic acid | 1 | 0.50-10 |
| Phenoxyethanol | 1 | 0.50-2.0 |
| Fragrances | 0.1 | 0.05-0.25 |
| PPG-26-Buteth-26 (and) PEG-40 Hydrogenated Castor Oil | 0.20 | 0.10-0.50 |

(continued)

| | w/w% | w/w% (range) |
|---|---|---|
| Water QS | 100 | 100 |
| pH QS with 20 % NaOH | 3.5 | 3.0-6.5 |

The untreated tresses and the treated tresses were washed with a non-conditioning shampoo of 10% SLS, 5, 10 and 15 times; the hair was dried after each wash. The Hunter color scale coordinates L, a, b were determined for each tress after 5, 10 and 15 washes and compared to the Hunter color scale coordinates L, a, b of the unwashed tresses.

The data are given below in Tables 6, 7 and 8 show significant color protection from the single treatment with the composition of the invention.

Table 6 - Untreated Versus Treated After 5 Washes

| Untreated 5 Washes | *L** | *a** | *b** | Δ*L** | Δ*a** | Δ*b** | Mean Δ*L** | Mean Δ*a** | Mean Δ*b** | Mean ΔE |
|---|---|---|---|---|---|---|---|---|---|---|
| LL1 5W | 22.27 | -13.68 | 0.9 | 4.71 | -7.14 | -1.37 | 4.74 | -7.53 | -1.13 | 9.00 |
| LL2 5W | 22.58 | -14.31 | 0.43 | 4.23 | -5.81 | -1.45 | | | | |
| LL3 5W | 21.68 | -12 | 0.34 | 5.27 | -9.64 | -0.57 | | | | |
| AVERAGE | 22.18 | -13.33 | 0.56 | 4.74 | -7.53 | -1.13 | | | | |
| | | | | | | | | | | |
| Treated 5 Washes | *L** | *a** | *b** | Δ*L** | Δ*a** | Δ*b** | Mean Δ*L** | Mean Δ*a** | Mean Δ*b** | Mean ΔE |
| LL1 5W | 22.16 | -13.93 | 0.58 | 3.61 | -5.37 | -1.78 | 2.89 | -4.35 | -1.54 | 5.45 |
| LL2 5W | 22.25 | -14.33 | 0.37 | 3.18 | -4.82 | -2.05 | | | | |
| LL3 5W | 20.61 | -11.25 | 0.42 | 1.88 | -2.86 | -0.78 | | | | |
| AVERAGE | 21.67 | -13.17 | 0.46 | 2.89 | -4.35 | -1.54 | | | | |

Table 7 - Untreated Versus Treated After 10 Washes

| Untreated 10 Washes | *L** | *a** | *b** | Δ*L** | Δ*a** | Δ*b** | Mean Δ*L** | Mean Δ*a** | Mean Δ*b** | Mean ΔE |
|---|---|---|---|---|---|---|---|---|---|---|
| LL1 10W | 21.68 | -12 | 0.34 | 5.51 | -8.55 | -1.56 | 6.33 | -9.18 | -2.09 | 11.39 |
| LL2 10W | 20.08 | -9.03 | -0.03 | 7.39 | -10.33 | -3.71 | | | | |
| LL3 10W | 23.55 | -15.05 | 1.14 | 6.08 | -8.67 | -1.01 | | | | |
| AVERAGE | 21.77 | -12.03 | 0.48 | 6.33 | -9.18 | -2.09 | | | | |
| | | | | | | | | | | |
| Treated 10 Washes | *L** | *a** | *b** | Δ*L** | Δ*a** | Δ*b** | Mean Δ*L** | Mean Δ*a** | Mean Δ*b** | Mean ΔE |
| LL1 10W | 20.74 | -11.68 | 0.49 | 4.07 | -7.5 | -2.75 | 3.31 | -6.03 | -2.32 | 7.29 |
| LL2 10W | 20.94 | -10.87 | 0.33 | 2.82 | -6.08 | -2.99 | | | | |
| LL3 10W | 20.84 | -11.45 | 0.58 | 3.04 | -4.51 | -1.21 | | | | |
| AVERAGE | 20.84 | -11.33 | 0.47 | 3.31 | -6.03 | -2.32 | | | | |

Table 8 - Treated Versus Untreated After 15 Washes

| Untreated 15 Washes | *L** | *a** | *b** | Δ*L** | Δ*a** | Δ*b** | Mean Δ*L** | Mean Δ*a** | Mean Δ*b** | Mean ΔE |
|---|---|---|---|---|---|---|---|---|---|---|
| LL1 15W | 20.08 | -9.03 | 0.03 | 6.32 | 9.26 | 4.21 | 5.59 | -7.19 | -2.80 | 9.56 |
| LL2 15W | 23.55 | -15.05 | 1.14 | 5.11 | 5.89 | 2.49 | | | | |
| LL3 15W | 21.88 | -13.34 | 0.91 | 5.35 | 6.42 | -1.7 | | | | |
| AVERAGE | 21.84 | -12.47 | 0.67 | 5.59 | -7.19 | -2.80 | | | | |

(continued)

| Untreated 15 Washes | *L** | *a** | *b** | *ΔL** | Δ*a** | Δ*b** | Mean Δ*L** | Mean Δ*a** | Mean Δ*b** | Mean ΔE |
|---|---|---|---|---|---|---|---|---|---|---|
| | | | | | | | | | | |
| Treated 15 Washes | *L** | *a** | *b** | *ΔL** | Δ*a** | Δ*b** | Mean Δ*L** | Mean Δ*a** | Mean Δ*b** | Mean ΔE |
| LL1 15W | 22.11 | -12.74 | 0.26 | 1.3 | 4.42 | 2.34 | 3.33 | -6.22 | -3.19 | 7.78 |
| LL2 15W | 21.69 | -14.01 | 0.69 | 4.34 | 7.55 | 3.65 | 3.33 | -6.22 | -3.19 | 7.78 |
| LL3 15W | 20.6 | -11 | 0.3 | 4.35 | 6.68 | 3.58 | | | | |
| AVERAGE | 21.47 | -12.58 | 0.42 | 3.33 | -6.22 | -3.19 | | | | |

Based on the ΔE of untreated and treated, after 5 washes the treated appears to have a 40 % protection of hair color. Where after 10 shampoos the treated tresses show a 36% color protection. Even after 15 shampoos there is measurable color protection of about 19%. These findings suggest the strong adsorption and hydrophobicity of the chitosan/vanillin crosslinked film on hair. These observations are in agreement with the detection of the film on the hair after 15 shampoos.

Example 12 - Preparation of a Treatment for Extremely Damaged Hair

A composition of the invention is prepared by combining the ingredients in the corresponding amounts listed in Table 9:

Table 9

| | w/w% | w/w% (range) |
|---|---|---|
| Chitosan | 1.0 | 0.01-2.0 |
| Vanillin | 0.250 | 0.00025-0.50 |
| Acetic acid | 0.500 | 0.50-10 |
| Diazolidinyl Urea(and)Iodopropynyl Butylcarbamate (and) Propylene Glycol | 0.400 | 0.40-2.0 |
| Fragrances | 0.100 | 0.05-0.25 |
| PPG-26-Buteth-26 (and) PEG-40 Hydrogenated Castor Oil | 0.200 | 0.10-0.50 |
| Magnesium sulfate | 0.025 | 0.10-2.0 |
| Glycerin | 5.000 | 0.5-10 |
| Water QS | 100.00 | |

The composition of Table 9 may be applied to severely damaged hair to improve hair feel, smoothness and the appearance of glossiness.

Example 13 - Preparation of Treatments To Improve Smoothness and Shine of Hair

Five compositions of the invention are prepared by combining the ingredients in the corresponding amounts listed in the Table 10 to 14, as shown below:

Table 10

| | w/w% | w/w% (range) |
|---|---|---|
| Chitosan | 1.0 | 0.01-2.0 |
| Vanillin | 0.250 | 0.00025-0.50 |
| Acetic acid | 0.500 | 0.50-10 |
| Diazolidinyl Urea(and)Iodopropynyl Butylcarbamate (and) Propylene Glycol | 0.400 | 0.40-2.0 |
| Fragrances | 0.100 | 0.05-0.25 |

(continued)

| | w/w% | w/w% (range) |
|---|---|---|
| PPG-26-Buteth-26 (and) PEG-40 Hydrogenated Castor Oil | 0.200 | 0.10-0.50 |
| Water QS | 100.00 | |

Table 11

| | w/w% | w/w% (range) |
|---|---|---|
| Chitosan | 0.500 | 0.01-2.0 |
| Vanillin | 0.125 | 0.0025-0.50 |
| Acetic acid | 0.500 | 0.50-2.0 |
| Diazolidinyl Urea(and)Iodopropynyl Butylcarbamate (and) Propylene Glycol | 0.400 | 0.4-2.0 |
| Fragrances | 0.100 | 0.05-0.25 |
| PPG-26-Buteth-26 (and) PEG-40 Hydrogenated Castor Oil | 0.200 | 0.10-0.50 |
| Trimethylsilocyamodimethicone and $C_{11-15}$ Pareth-7 and $C_{12-16}$ Pareth-9 and Glycerine and Trideceth-12 | 1.00 | 0.10-3.0 |
| Water QS | 100.00 | |

Table 12

| | w/w% | w/w% (range) |
|---|---|---|
| Chitosan | 0.500 | 0.01-2.0 |
| Vanillin | 0.125 | 0.0025-0.50 |
| Acetic acid | 0.500 | 0.50-2.0 |
| Diazolidinyl Urea(and)Iodopropynyl Butylcarbamate (and) Propylene Glycol | 0.400 | 0.40-2.0 |
| Fragrances | 0.100 | 0.05-0.25 |
| PPG-26-Buteth-26 (and) PEG-40 Hydrogenated Castor Oil | 0.200 | 0.10-0.50 |
| Polyquaternium-11 (and) Water | 0.500 | 0.10-3.0 |
| Water QS | 100.000 | |

Table 13

| | w/w% | w/w% (range) |
|---|---|---|
| Chitosan | 0.500 | 0.01-2.0 |
| Vanillin | 0.125 | 0.0025-0.50 |
| Acetic acid | 0.500 | 0.50-2.0 |
| Diazolidinyl Urea(and)Iodopropynyl Butylcarbamate (and) Propylene Glycol | 0.400 | 0.40-2.0 |
| Fragrances | 0.100 | 0.05-0.25 |
| PPG-26-Buteth-26 (and) PEG-40 Hydrogenated Castor Oil | 0.200 | 0.10-0.50 |
| Polyquaternium-55 (and) Water | 0.500 | 0.10-3.0 |
| Water QS | 100.000 | |

Table 14

| | w/w% | w/w% (range) |
|---|---|---|
| Chitosan | 0.500 | 0.01-2.0 |

(continued)

| | w/w% | w/w% (range) |
|---|---|---|
| Vanillin | 0.125 | 0.0025-0.50 |
| Acetic acid | 0.500 | 0.50-2.0 |
| Diazolidinyl Urea(and)Iodopropynyl Butylcarbamate (and) Propylene Glycol | 0.400 | 0.40-2.0 |
| Fragrances | 0.100 | 0.05-0.25 |
| PPG-26-Buteth-26 (and) PEG-40 Hydrogenated Castor Oil | 0.200 | 0.10-0.50 |
| Polyquaternium-6 (and) Water | 1.00 | 0.10-3.0 |
| Water QS | 100.000 | |

The compositions of any of Tables 10 to 14 may be applied to hair which is then blow dried to form a film on the hair that improves the hair's appearance of smoothness and shine.

Example 14 - Preparation of "Leave In" Treatment for Extremely Damaged Hair

A composition of the invention is prepared by combining the ingredients in the corresponding amounts listed in Table 15:

Table 15

| | w/w% | w/w% (range) |
|---|---|---|
| Chitosan | 0.40 | 0.01-4.0 |
| Vanillin | 0.10 | 0.04-1.0 |
| Zinc acetate | 0.05 | 0.0013-0.50 |
| Acetic acid | 1 | 0.50-10 |
| Phenoxyethanol | 1 | 0.50-2.0 |
| Fragrance | 0.1 | 0.05-2.0 |
| PG-26-Buteth-26 (and) PEG-40 Hydrogenated Castor Oil | 0.20 | 0.10-0.50 |
| Water QS | 100 | 100 |
| pH QS with 20% NaOH | 3.5 | 3.0-6.5 |

The effect of this composition on the tensile strength of hair was evaluated.

Hair was treated as in Example 8, where normal hair was processed for five minutes, dried under the dryer and then rinsed. The tensile strength of hair before and after treatment is shown in Table 16 below.

Table 16 - Average Load (g)

| Strain | Untreated; n=60 fibers | Treated; n=60 fibers | | % Strength Increase |
|---|---|---|---|---|
| 2% | 49.35 | 79.65 | p=0.0533 | 61.40 |
| 10% | 234.37 | 263.02 | p=0.00188 | 12.22 |
| 20% | 259.41 | 294.61 | p=0.00916 | 13.57 |

Strength Increase = (Avg Load Treated - Avg Load Untreated)/ Avg Load Untreated x 100

The composition has improved the tensile strength of normal hair. The increase in wet tensile strength statistically is in the elastic and the yield region. The increase in the elastic region strength is about 60% and about 12-14% in the yield region. This increase in tensile strength is probably due to an increase in the matrix viscosity with the formed chitosan/vanillin imino Schiff base.

Example 15 - Preparation of Hair Gels That are Capable of Providing the Film of the Invention

Three hair gels were prepared, each capable of forming the film of the invention when applied to hair. Each gel was prepared by combining the ingredients/amounts as shown in the Tables 17 to 19, below.

Table 17

| | w/w % | w/w % Range |
|---|---|---|
| Chitosan | 0.60 | 0.01-2.00 |
| Vanillin | 0.15 | 0.0025-0.50 |
| Acetic Acid | 1.00 | 0.50-3.0 |
| Diazolidinyl Urea (and) Iodopropynyl Butylcarbamate (and) Propylene Glycol | 0.40 | 0.40-2.00 |
| Arginine | 1.20 | 0.50-2.00 |
| Fragrance | 0.10 | 0.10-0.50 |
| PPG-26-Buteth-26 (and) PEG-40 Hydrogenated Castor Oil (and) Water | 0.20 | 0.10-3.0 |
| Water QS | 100 | |
| pH QS Arginine | 6.00 | 3.50-6.00 |

Table 18

| | w/w % | w/w % Range |
|---|---|---|
| Chitosan | 0.60 | 0.01-2.00 |
| Vanillin | 0.15 | 0.0025-0.50 |
| Acetic Acid | 1.00 | 0.50-3.0 |
| Diazolidinyl Urea (and) Iodopropynyl Butylcarbamate (and) Propylene Glycol | 0.40 | 0.40-2.00 |
| Sodium Hydroxide | 0.70 | 0.50-2.00 |
| Fragrance | 0.10 | 0.10-0.50 |
| PPG-26-Buteth-26 (and) PEG-40 Hydrogenated Castor Oil (and) Water | 0.20 | 0.10-3.0 |
| Water QS | 100 | |
| pH QS NaOH | 6.00 | 3.50-6.00 |

Table 19

| | w/w % | w/w % Range |
|---|---|---|
| Chitosan | 0.60 | 0.01-2.00 |
| Vanillin | 0.15 | 0.0025-0.5 |
| Acetic Acid | 1.00 | 0.50-3.0 |
| Diazolidinyl Urea (and) Iodopropynyl Butylcarbamate (and) Propylene Glycol | 0.40 | 0.40-2.00 |
| Ammonium Hydroxide | 0.25 | 0.10-2.00 |
| Fragrance | 0.10 | 0.10-0.50 |
| PPG-26-Buteth-26 (and) PEG-40 Hydrogenated Castor Oil (and) Water | 0.20 | 0.10-3.0 |
| Water QS | 100 | |
| pH QS $NH_4OH$ | 6.00 | 3.50-6.00 |

Example 16 - Evaluation of the Composition on the Diameter of Hair

The effect of the composition of Example 5 on the geometry of hair has been evaluated on normal fine < 70 um and

20 volume color treated hair.

The mean diameter of ten fibers was determined using the Diastron, Mitutoyo LSM-5000 laser micrometer. The mean diameter measurement of ten slices or points on the fiber is determined. The fibers were treated as per Example 8 and dried under the dryer, rinsed and shampooed in between treatments.

Table 20 shows below the data of the mean diameter before and after the treatment.

Table 20

| | Diameter Untreated (um) | Diameter One treatment (um) | Diameter Percent Increase | Diameter Three Treatments (um) | Diameter Percent Increase | Diameter Six Treatments (um) | Diameter Percent Increase** |
|---|---|---|---|---|---|---|---|
| Normal fine hair | 65.88 | 68.63 | 4.17 p=0.0047 | 70.99 | 7.76 p=0.0045 | 75.01 | 13.86 p=0.0041 4 |
| 20 volume colored hair | 70.28 | 72.95 | 3.80 p=0.005 | | | 79.05 | 12.48 p=0.0000 477 |

** % Increase Diameter= (Diameter Treatment-Diameter Untreated) / Diameter Untreated x 100

The data in Table 20 show that the mean diameter of hair is increased by about 4% from a single treatment and to 13.86% after six treatments and six washings. It also establishes that multiple treatments can progressively increase the diameter of hair and shampooing does not easily leach the chitosan:aldehyde polymer out of hair. The treatment visually thickens, volumizes hair with shine, luster enhancement, and minimum static charge and frizz. The hair appears fuller with healthy movement.

Based on the tensile strength data and washings, it appears that the Chitosan-aldehyde Schiff base polymer film does improve the mechanical properties of hair. The hair appears thicker, moisturized and conditioned. This effect is surprising, since conventionally conditioned and moisturized hair has less body and volume.

Example 17 - Demonstration of Strong Affinity and Adherence of Crosslinked Film of the Invention on Human Hair

An evaluation was carried out to demonstrate the strong affinity and adherence of the film of the Invention to human hair, even after only a single treatment. The method used for evaluation was a staining method described below and in further detail in in R. J. Crawford and C. R. Robbins, A replacement for Rubine dye for detecting cationics on keratin, J. Soc. Cosmet, Chem., 31, 273-278 (1980), the contents of which are incorporated herein by reference. This method uses the dye Red 80.

To carry out the evaluation, the Hunter color coordinates L, a, b of untreated 100 % white hair tresses were measured. Subsequently, the hair tresses were treated with a composition of the ingredients/amounts set out in Table 21:

Table 21

| | w/w% | w/w% (range) |
|---|---|---|
| Chitosan | 0.6 | 0.01-2.0 |
| Vanillin | 0.15 | 0.00025-0.50 |
| Acetic acid | 1.0 | 0.50-10 |
| Phenoxyethanol | 1 | 0.50-2.0 |
| Fragrances | 0.1 | 0.05-0.25 |
| PPG-26-Buteth-26 (and) PEG-40 Hydrogenated Castor Oil | 0.20 | 0.10-0.50 |
| Water QS | 100 | 100 |
| pH QS with 20 % NaOH | 3.5 | 3.0-6.5 |

The tresses were then dried with a blow dryer at a high heat setting.

Once dry, the untreated and treated tresses were immersed into 50 ml of a solution of 0.5% Red 80 and 0.125% acetic acid for 60 seconds. The tresses were removed and rinsed with tepid water for 2 minutes, blotted and thoroughly dried using a blow dryer. The level of staining or adsorption of Red 80 on untreated and treated hair tresses was evaluated by measuring the hunter color coordinates L, a, b initially before shampooing (0 shampoos), and after 5, 10, and 15 shampoos, each using a non-conditioning shampoo. The tresses were dried to about 80% between shampoos.

The results are shown below in Table 22.

Table 22

| | L | a | b | L* | a* | ΔL | Δa | Δb | ΔE |
|---|---|---|---|---|---|---|---|---|---|
| Untreated | 71.58 | 2.52 | 23.24 | 71.11 | 3.72 | -0.47 | 1.2 | -1.5 | 1.98 |
| Untreated 15 Shampoos | 72.63 | 3.65 | 24.10 | 73.32 | 5.36 | 0.69 | 1.71 | -0.67 | 2.06 |
| 0 Shampoos | 70.26 | 3.73 | 24.13 | 54.26 | 31.97 | - 16.38 | 28.24 | -9.89 | 34.12 |
| 5 shampoos | 71.13 | 2.49 | 24.17 | 61.08 | 20.21 | - 10.05 | 17.72 | -9.34 | 22.42 |
| 10 Shampoos | 71.77 | 2.61 | 24.27 | 62.21 | 18.91 | -9.56 | 16.30 | -9.61 | 21.21 |
| 15 Shampoos | 71.56 | 2.68 | 24.62 | 66.32 | 13.38 | -5.24 | 10.70 | -6.83 | 13.73 |
| * White hair dyed with Red 80 as per directions. | | | | | | | | | |

As can be seen from the data above, the untreated hair did not absorb any dye based on the Δa of 1.2 indicating that no cationics were present on the hair. This indicates an absence of the film of the invention on the hair or any other cationics, as would be expected. However, the hair tresses that were subjected to the single chitosan/vanillin treatment shows the presence of a strong cationic charge on hair of Δa of 28.24 (indicating the presence of the film resulting from the chitosan/vanillin treatment) and a resistance of removal of this film by shampooing. The adsorption, affinity or strong bonding of the chitosan/vanillin film on hair lasts at least 15 shampoos, as is shown primarily from the persistence of a values. Even after 15 shampoos the Δa is 10.70 which shows about 38% of the film is still on the hair tresses. This strong cationic character of hair is responsible for the long lasting conditioning effects detected by wet combing, where resistance to combing (and tangling) is minimal. Untreated tresses, even after 15 shampoos, show no adsorption of the Red 80. Thus, one concludes that contribution to the staining is due to the chitosan/vanillin Schiff base and film formation on hair.

Example 18 - Use of the Film of the Invention to Increase the Volume of Fine Hair

The effect of the crosslinked chitosan/vanillin film on the volume of fine hair was evaluated using a polarization imaging method described below and in more detail by N. Lechochinski and S.Breugnot , J.Cosmet.Sci, (2011).

A composition to treat fine hair was prepared by mixing the ingredients/amounts as shown below in Table 23.

Table 23

| | w/w% |
|---|---|
| Chitosan | 0.600 |
| Vanillin | 0.150 |
| Acetic acid | 1.00 |
| Diazolidinyl Urea(and)Iodopropynyl Butylcarbamate (and) Propylene Glycol | 0.400 |
| Fragrances | 0.100 |
| PPG-26-Buteth-26 ( and) PEG-40 Hydrogenated Castor Oil | 0.200 |
| Water QS | 100.00 |

To prepare hair bundles for analysis, 8 inch long hair bundles (9 grams each, from Fine Hair, International Hair Importers) were obtained and were washed with 10% SLES solution by applying 1 ml of SLES solution to the hair, agitating for 60 seconds and rinsing for 30 seconds under tap water set at 38°C (+/- 2.5°C) with a flow rate of 60 ml/sec. Each bundle was then completely immersed in a beaker of warm water and swirled for 60 seconds.

The bundles were removed from the beaker and dried by first squeezing out excess water by running bundle between ring finger and index finger, then blotting the bundle by pressing between two paper towels. The bundles were

combed to remove any snarls with a fine toothed comb. The bundles were each hung on a ring stand, positioned so that the top of the bundle was flush with the top of a 3 prong clamp. A SHARPIE® marker was used to mark the area on the bundle above the middle prong to identify the precise position of the bundle in the clamp throughout the test.

A hair dryer was set on high heat and high speed, and the bundles each were dried in the clamp by applying the hair dryer and a vent brush in a downward motion. While drying, the bundle was "trapped" between the blow dryer and the brush, and the brush pulled straight down and through the entire bundle for 15 second durations on each side, repeating until a total of 3 minutes of drying time had passed. Subsequently, the dryer was positioned underneath the bundle, allowing the bundle to hang freely and the dryer was maintained in an upward motion to dry the very top of the bundle for 10 additional seconds, being sure not to burn the wax seal securing the individual hairs of the bundle together.

Each bundle was removed from the clamp, any snarls were brushed out and the bundle was hung on a bundle board.

Once this process was carried out on all the bundles, the bundles were placed into a humidity chamber (set at 30°C and 50% R.H.) to allow any static in the bundles to dissipate. Bundles were left in the humidity room for 3 hours before any measurements were taken.

Each bundle was then hung in the RUMBA holder in the exact position it was during the initial blow drying step. RUMBA and BOLERO volume measurements were taken for each bundle prior to treatment.

After hanging overnight, each bundle was treated as follows. Each was swirled in a beaker of warm water for 60 seconds, making sure that the entire bundle was immersed and saturated. Upon removal from beaker, the excess water was removed from the bundles by running hairs of the bundle between fingers. To each bundle was applied 1 ml of the chitosan/vanillin composition of Table 10A; the composition was massaged in to the hairs to evenly distribute and the bundle was allowed to sit for a total of 1 minute.

The bundles were blow dried and left in the humidity chamber as described above. Once removed from the humidity chamber, RUMBA and BOLERO volume measurements of the treated bundles were taken.

The percent change was calculated for all measurements and an average percent decrease/increase calculated; the data are set forth in Table 40, below:

Table 24

| Bundle | Baseline Measurement (Untreated) in $mm^3$ | Treated Measurement (Treatment + Pre-Treatment) $mm^3$ | % Volume Increase |
|---|---|---|---|
| A | 2.03 | 3.37 | 66.01 |
| B | 2.66 | 4.01 | 50.75 |
| C | 2.59 | 3.62 | 39.77 |
| P | | | 0.0071 |
| Standard Error | 0.20 | 0.16 | |
| Standard deviation | 0.30 | .024 | |
| | | % volume increase | 44.87 |

The volume of hair increased by 45%, p=0.0071, from a single treatment.

Example 19 - A Volumizing Hair Treatment for Normal Fine and Chemically Damaged Hair

A composition is prepared by mixing the ingredients and amounts set forth in Table 25.

Table 25

| | w/w% | w/w% (range) |
|---|---|---|
| Chitosan | 0.40 | 0.01-4 |
| Vanillin | 0.10 | 0.04-1 |
| Glycerol | 0.50 | 0.20-40 |
| Acetic acid | 1 | 0.50-10 |
| Phenoxyethanol | 1 | 0.50-2 |
| Fragrance | 0.1 | 0.05-0.30 |

(continued)

| | w/w% | w/w% (range) |
|---|---|---|
| PPG-26-buteth-26 (and) PEG-40 hydrogenated castor oil | 0.20 | 0.10-0.50 |
| Water QS | 100 | 100 |
| pH QS with 20% NaOH | 4.5 | 4.0-8.9 |

The composition prepared and evaluated in this example is specific for volumizing or bodifying hair. The addition of low levels of glycol and polyglycols such as glycerol, propylene glycol, ethylene glycol tend to affect the characteristics of the chitosan:aldehyde polymer films. It appears that the stiffness decreases and the pliability increases in the presence of glycols. This appears to have a softening effect. The chitosan: aldehyde: glycerine films are soft and appear to be thicker and have an occlusive behavior towards water.

At concentrations of glycol at about 30-35%, chitosan 1%, vanillin 0.25% and acetic acid 1% and pH between 5-7 liquid aqueous crystal gel solutions are formed. These gel compositions have additional benefits on hair beyond volumizing.

Example 20 - Preparation and Evaluation of Volumizing Benefit of Anionic Complex Coacervate With Chitosan: Aldehyde

An anionic complex coacervate with chitosan: aldehyde was prepared and its effect on hair volume was evaluated. An anionic formulation was prepared using sodium polystyrene sulfonate as an (poly)anionic material by incorporating the following ingredients together in the corresponding amounts listed in Table 26:

Table 26 - Anionic Formulation

| | w/w % |
|---|---|
| Sodium Polystyrene Sulfonate | 1.00 |
| Diazolidinyl Urea (and) Iodopropynyl Butylcarbamate (and) Propylene Glycol | 0.50 |
| Water QS | 100 |
| pH | 7.00 |

A chitosan and vanillin formulation was prepared by incorporating the following ingredients together in the corresponding amounts listed in Table 27:

Table 27

| | w/w % |
|---|---|
| Chitosan | 0.60 |
| Vanillin | 0.15 |
| Acetic Acid | 1.00 |
| Diazolidinyl Urea (and) Iodopropynyl Butylcarbamate (and) Propylene Glycol | 0.40 |
| Fragrance | 0.10 |
| PPG-26-Buteth-26 (and) PEG-40 Hydrogenated Castor Oil (and) Water | 0.20 |
| Water QS | 100 |
| pH QS | 4.00 |

To apply the complex coacervate to hair, human head hair *in situ* (*i.e.,* on head) was first shampooed with a clarifying shampoo, and towel dried by blotting to remove excess water. The anionic formulation of Table 26 was applied to the root sections of hair (~ 2 inches from scalp) and allowed to process for about 1 minute. Subsequently, the chitosan and vanillin composition of Table 27 was applied to hair from root to ends.

The hair was dried with a blow dryer to about 80% dryness.

At 80% dryness, the hair is blow dried using a hair dryer and a hair brush, where the hair is wrapped around the

brush once entirely, in an upward motion beginning from the hair roots.

Once the hair root area, shaft and ends are dry, the hair is sectioned and each section is cooled by the application of cool air until completely cooled.

Increase in volume of the hair was evaluated using the using the polarization imaging method described above and in detail by N. Lechochinski and S. Breugnot, J.Cosmet.Sci, March/April 2011. The polyanion, polystyrene sulfonate complex coacervate formation with chitosan/vanillin on hair and its effect on tresses using the procedure above demonstrates good volume increase from a single treatment that lasted 5-6 shampoos or more. The volume increase was analyzed as 75.19% ( p= 0.0083).

This volumizing effect was confirmed further with salon half head testing of nine models. The initial volume effects included root lift, more side and frontal volume. These effects did not diminish during the entire day and no support styling products were necessary to maintain or preserve the volumizing effect.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the scope of the present invention as defined by the appended claims.

## Claims

1. A method of forming a semi-permanent film on a hair fiber comprising topically applying an effective amount of a composition to the hair and exposing the hair to a temperature of 60°C to 190°C thereby forming a film on the hair, wherein the composition comprises a chitosan, an aldehyde-bearing compound, and an organic acid, wherein the aldehyde-bearing compound is selected from a phenolic aldehyde.

2. The method of claim 1, wherein the fiber is dampened with an aqueous solvent.

3. The method of claim 1, wherein the composition further includes an aqueous solvent.

4. The method of claim 1, wherein the chitosan of the composition is selected from (i) chitosan having a molecular weight of 50,000 to 1,000,000 g/mole and 95% deacetylation (ii) 300,000 to 2,000,000 g/mole and 95 % deacetylation and (iii) chitosan having a molecular weight of 500,000 to 5,000,000 g/mole and 80% deacetylation.

5. The method of claim 1, wherein the aldehyde-bearing compound is selected from vanillin and p-vanillin.

6. The method of claim 1, wherein the fiber is exposed to a temperature of about 100°C to about 160°C.

7. A composition capable of forming a semi-permanent film in situ on a fiber in the presence of heat of a temperature of 60 °C to 190°C comprising a chitosan, an aldehyde-bearing compound, and an organic acid, wherein the aldehyde-bearing compound is selected from a phenolic aldehyde.

8. The composition of claim 7 wherein, the chitosan of the composition is selected from (i) chitosan having a molecular weight of 50,000 to 1,000,000 g/mole and 95% deacetylation, (ii) 300,000 to 2,000,000 g/mole and 95% deacetylation and (iii) chitosan having a molecular weight of 500,000 to 5,000,000 g/mole and 80% deacetylation.

9. The composition of claim 7 wherein the aldehyde-bearing compound is selected from vanillin and p-vanillin.

10. A method of semi-permanently altering the structure of a hair comprising topically applying an effective amount of the composition of any of claims 7 - 9 to the hair and subsequently exposing the hair to a temperature of 60°C to 190°C to form a film on the hair.

11. The method of claim 10, wherein the film persists on the hair after 3-20 washings.

## Patentansprüche

1. Verfahren zur Bildung eines semipermanenten Films auf einer Haarfaser, umfassend
das topische Auftragen einer wirksamen Menge einer Zusammensetzung auf das Haar und das Aussetzen des Haares einer Temperatur von etwa 60 °C bis etwa 190 °C, wodurch ein Film auf dem Haar gebildet wird, wobei die

Zusammensetzung ein Chitosan, eine aldehydhaltige Verbindung und eine organische Säure umfasst, wobei die aldehydhaltige Verbindung aus einem phenolischen Aldehyd ausgewählt ist.

**2.** Verfahren nach Anspruch 1, wobei die Haarfaser mit einem wässrigen Lösungsmittel befeuchtet wird.

**3.** Verfahren nach Anspruch 1, wobei die Zusammensetzung ferner ein wässriges Lösungsmittel enthält.

**4.** Verfahren nach Anspruch 1, wobei das Chitosan der Zusammensetzung ausgewählt ist aus (i) Chitosan mit einem Molekulargewicht von 50.000 bis 1.000.000 g/mol und einem Deacetylierungsgrad von 95 % (ii) 300.000 bis 2.000.000 g/mol und einem Deacetylierungsgrad von 95 % sowie (iii) Chitosan mit einem Molekulargewicht von 500.000 bis 5.000.000 g/mol und einem Deacetylierungsgrad von 80 % ausgewählt ist.

**5.** Verfahren nach Anspruch 1, wobei die aldehydhaltige Verbindung aus Vanillin und p-Vanillin ausgewählt ist.

**6.** Verfahren nach Anspruch 1, wobei die Faser einer Temperatur von etwa 100 °C bis etwa 160 °C ausgesetzt wird.

**7.** Zusammensetzung, die in der Lage ist, in situ auf einer Faser in Gegenwart von Wärme mit einer Temperatur von etwa 60 °C bis etwa 190 °C einen semipermanenten Film zu bilden, umfassend Chitosan, eine aldehydhaltige Verbindung und eine organische Säure, wobei die aldehydhaltige Verbindung aus einem phenolischen Aldehyd ausgewählt ist.

**8.** Zusammensetzung nach Anspruch 7, wobei das Chitosan der Zusammensetzung ausgewählt ist aus (i) Chitosan mit einem Molekulargewicht von 50.000 bis 1.000.000 g/mol und einem Deacetylierungsgrad von 95 %, (ii) Chitosan mit einem Molekulargewicht von 300.000 bis 2.000.000 g/mol und einem Deacetylierungsgrad von 95 % sowie (iii) Chitosan mit einem Molekulargewicht von 500.000 bis 5.000.000 g/mol und einem Deacetylierungsgrad von 80 %.

**9.** Zusammensetzung nach Anspruch 7, wobei die aldehydhaltige Verbindung aus Vanillin und p-Vanillin ausgewählt ist.

**10.** Verfahren zur semipermanenten Veränderung der Struktur eines Haares, umfassend das topische Auftragen einer wirksamen Menge der Zusammensetzung gemäß einem der Ansprüche 7 bis 9 auf das Haar und das anschließende Aussetzen des Haares einer Temperatur von etwa 60 °C bis etwa 190 °C, um einen Film auf dem Haar zu bilden.

**11.** Verfahren nach Anspruch 10, wobei der Film nach 3-20 Haarwäschen auf dem Haar bestehen bleibt.

## Revendications

**1.** Procédé de formation d'un film semi-permanent sur une fibre capillaire, comprenant
l'application topique d'une quantité efficace d'une composition sur le cheveu et l'exposition du cheveu à une température comprise entre environ 60 °C et environ 190 °C, formant ainsi un film sur le cheveu, dans lequel la composition comprend du chitosane, un composé contenant un aldéhyde et un acide organique, le composé contenant un aldéhyde étant choisi parmi un aldéhyde phénolique.

**2.** Procédé selon la revendication 1, dans lequel la fibre est humidifiée avec un solvant aqueux.

**3.** Procédé selon la revendication 1, dans lequel la composition comprend en outre un solvant aqueux.

**4.** Procédé selon la revendication 1, dans lequel le chitosane de la composition est choisi parmi (i) un chitosane ayant un poids moléculaire compris entre 50 000 et 1 000 000 g/mole et un taux de désacétylation de 95 % (ii) un poids moléculaire compris entre 300 000 et 2 000 000 g/mole et un taux de désacétylation de 95 % ; et (iii) un chitosane ayant un poids moléculaire compris entre 500 000 et 5 000 000 g/mole et un taux de désacétylation de 80 %.

**5.** Procédé selon la revendication 1, dans lequel le composé contenant un groupe aldéhyde est choisi parmi la vanilline et la p-vanilline.

**6.** Procédé selon la revendication 1, dans lequel la fibre est exposée à une température comprise entre environ 100 °C et environ 160 °C.

**7.** Composition capable de former in situ un film semi-permanent sur une fibre en présence de chaleur à une température

comprise entre environ 60 °C et environ 190 °C, comprenant du chitosane, un composé contenant un groupe aldéhyde et un acide organique, dans laquelle le composé contenant un groupe aldéhyde est choisi parmi un aldéhyde phénolique.

**8.** Composition selon la revendication 7, dans laquelle le chitosane de la composition est

choisi parmi (i) un chitosane ayant un poids moléculaire compris entre 50 000 et 1 000 000 g/mole et un taux de désacétylation de 95 %, (ii) un chitosane ayant un poids moléculaire compris entre 300 000 et 2 000 000 g/mole et un taux de désacétylation de 95 %, et
(iii) un chitosane ayant un poids moléculaire compris entre 500 000 et 5 000 000 g/mole et un taux de désacétylation de 80 %.

**9.** Composition selon la revendication 7, dans laquelle le composé contenant un groupe aldéhyde est choisi parmi la vanilline et la p-vanilline.

**10.** Procédé permettant de modifier de manière semi-permanente la structure d'un cheveu, comprenant l'application topique d'une quantité efficace de la composition selon l'une quelconque des revendications 7 à 9 sur le cheveu, puis l'exposition du cheveu à une température comprise entre environ 60 °C et environ 190 °C afin de former un film sur le cheveu.

**11.** Procédé selon la revendication 10, dans lequel le film persiste sur le cheveu après 3 à 20 lavages.

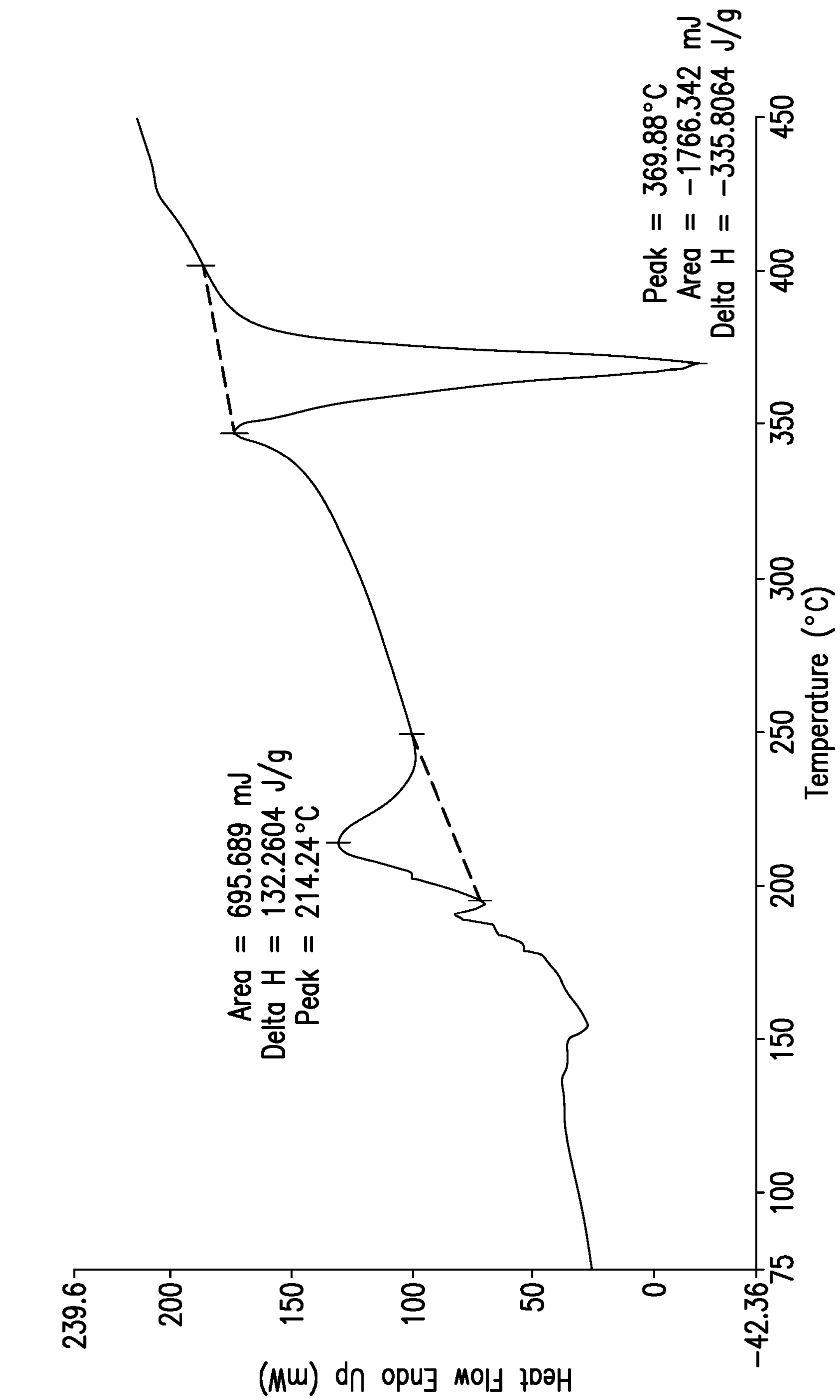
Area = 695.689 mJ
Delta H = 132.2604 J/g
Peak = 214.24°C
Peak = 369.88°C
Area = −1766.342 mJ
Delta H = −335.8064 J/g
Heat Flow Endo Up (mW)
239.6
200
150
100
50
0
−42.36
75
100
150
200
250
300
350
400
450
Temperature (°C)

FIG.1

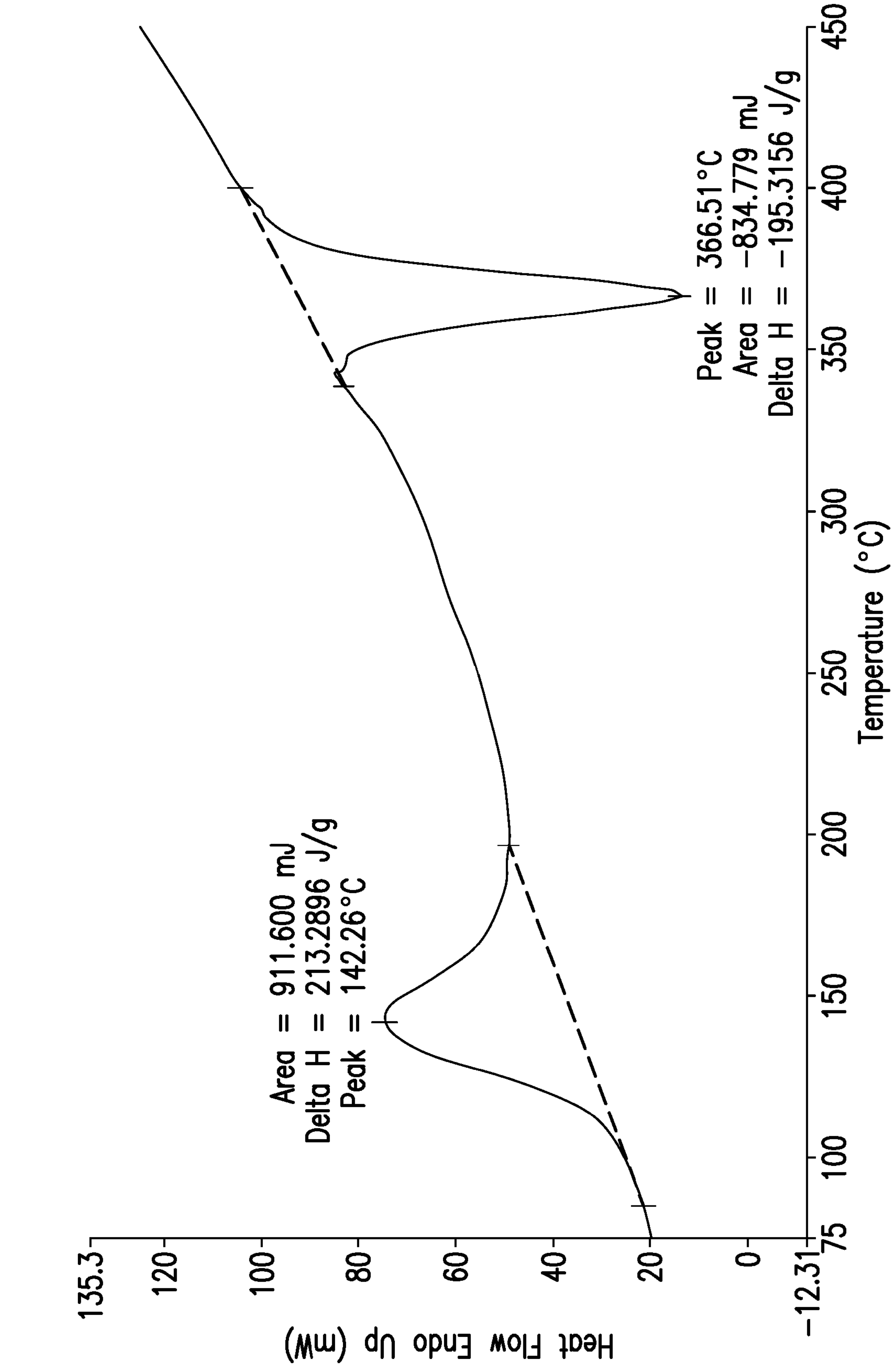
Heat Flow Endo Up (mW)
135.3
120
100
80
60
40
20
0
−12.31
75
100
150
200
250
300
350
400
450
Temperature (°C)
Area = 911.600 mJ
Delta H = 213.2896 J/g
Peak = 142.26°C
Peak = 366.51°C
Area = −834.779 mJ
Delta H = −195.3156 J/g

FIG.2

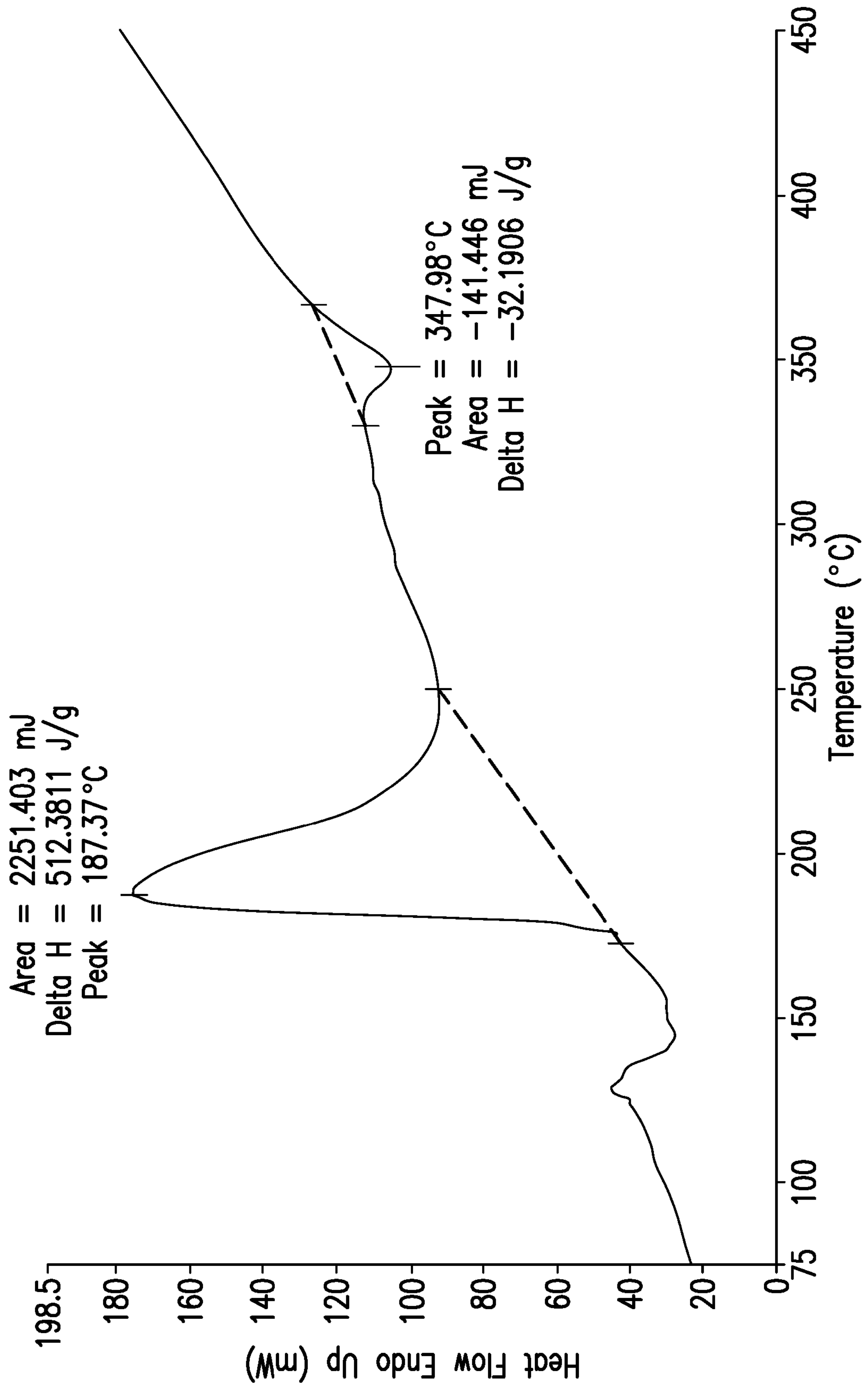
Area = 2251.403 mJ
Delta H = 512.3811 J/g
Peak = 187.37°C
Peak = 347.98°C
Area = −141.446 mJ
Delta H = −32.1906 J/g
Heat Flow Endo Up (mW)
198.5
180
160
140
120
100
80
60
40
20
0
75
100
150
200
250
300
350
400
450
Temperature (°C)

FIG.3

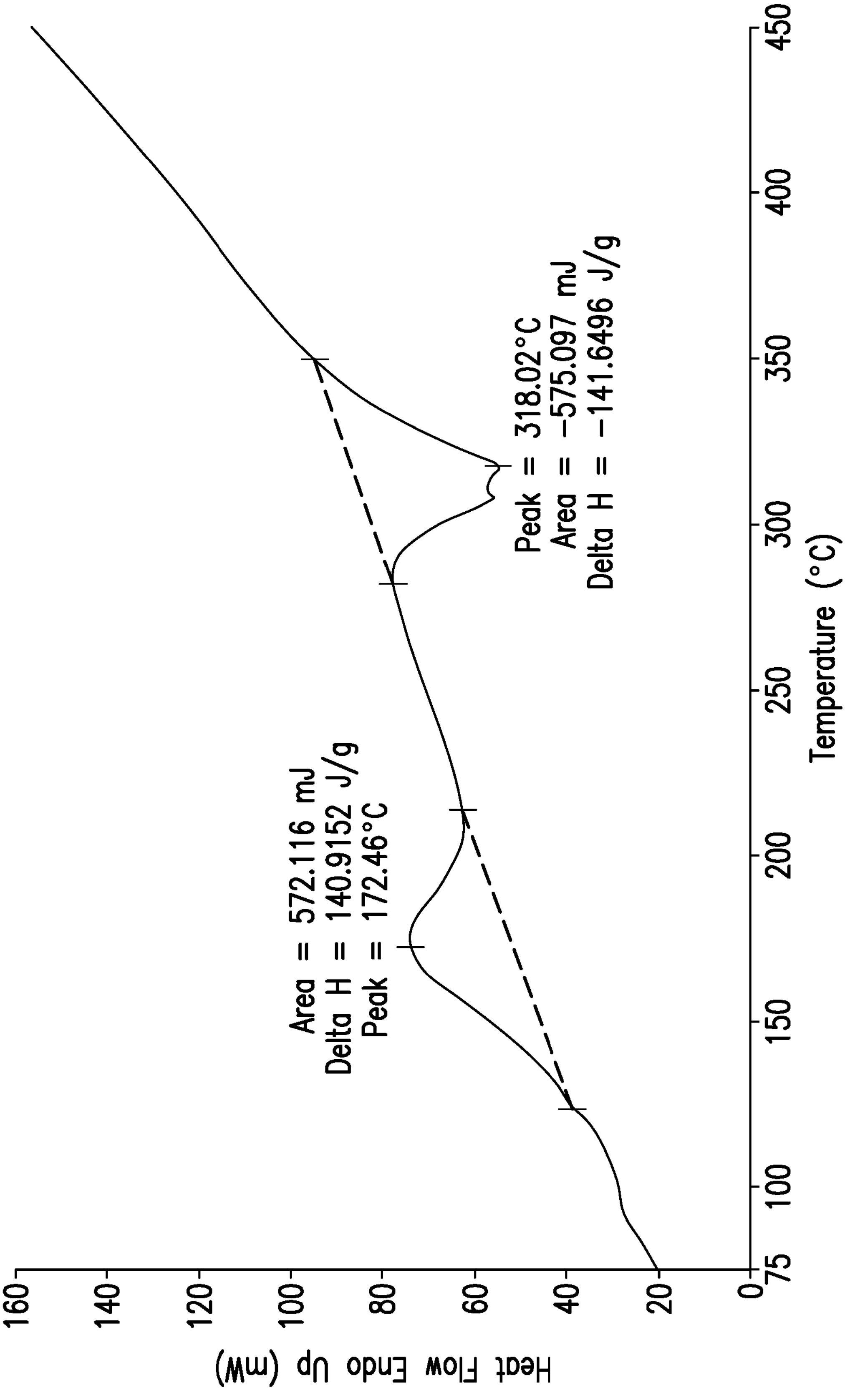
Heat Flow Endo Up (mW)
0
20
40
60
80
100
120
140
160
Temperature (°C)
75
100
150
200
250
300
350
400
450
Area = 572.116 mJ
Delta H = 140.9152 J/g
Peak = 172.46°C
Peak = 318.02°C
Area = −575.097 mJ
Delta H = −141.6496 J/g

FIG.4

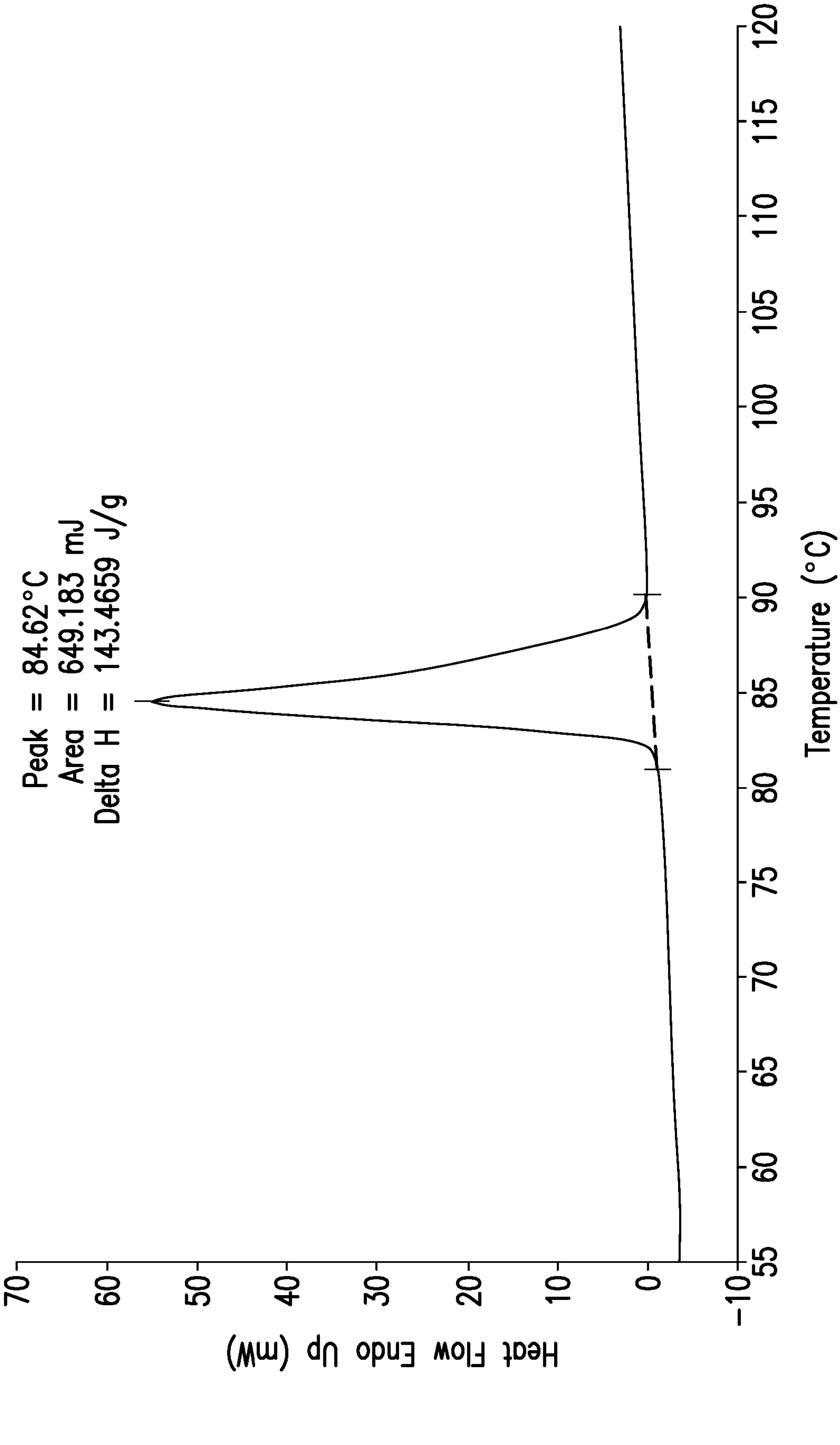
Peak = 84.62°C
Area = 649.183 mJ
Delta H = 143.4659 J/g
Heat Flow Endo Up (mW)
70
60
50
40
30
20
10
0
–10
55
60
65
70
75
80
85
90
95
100
105
110
115
120
Temperature (°C)

FIG.5

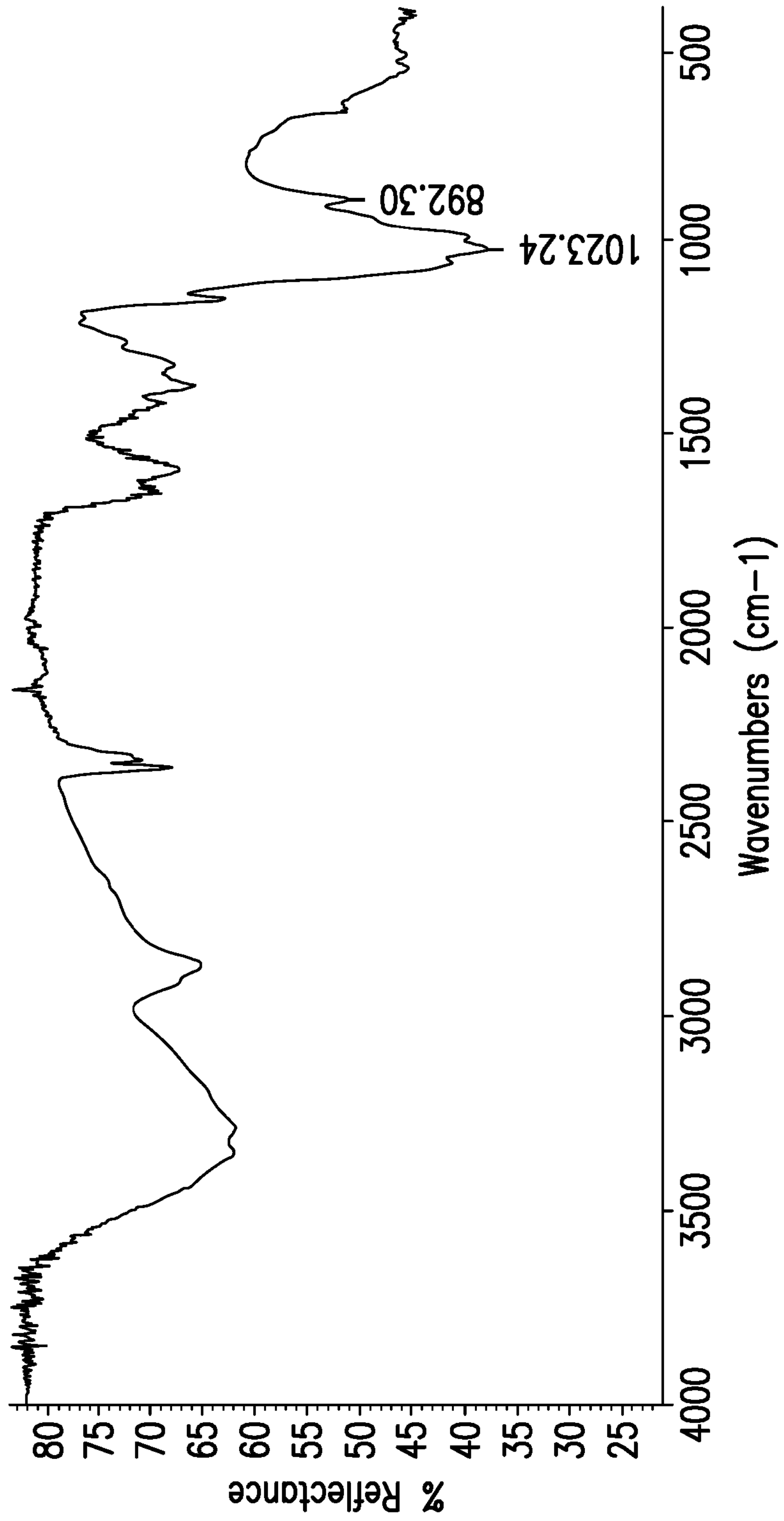
Chitosan Powder
% Reflectance
80
75
70
65
60
55
50
45
40
35
30
25
4000
3500
3000
2500
2000
1500
1000
500
Wavenumbers (cm−1)
1023.24
892.30

FIG.6

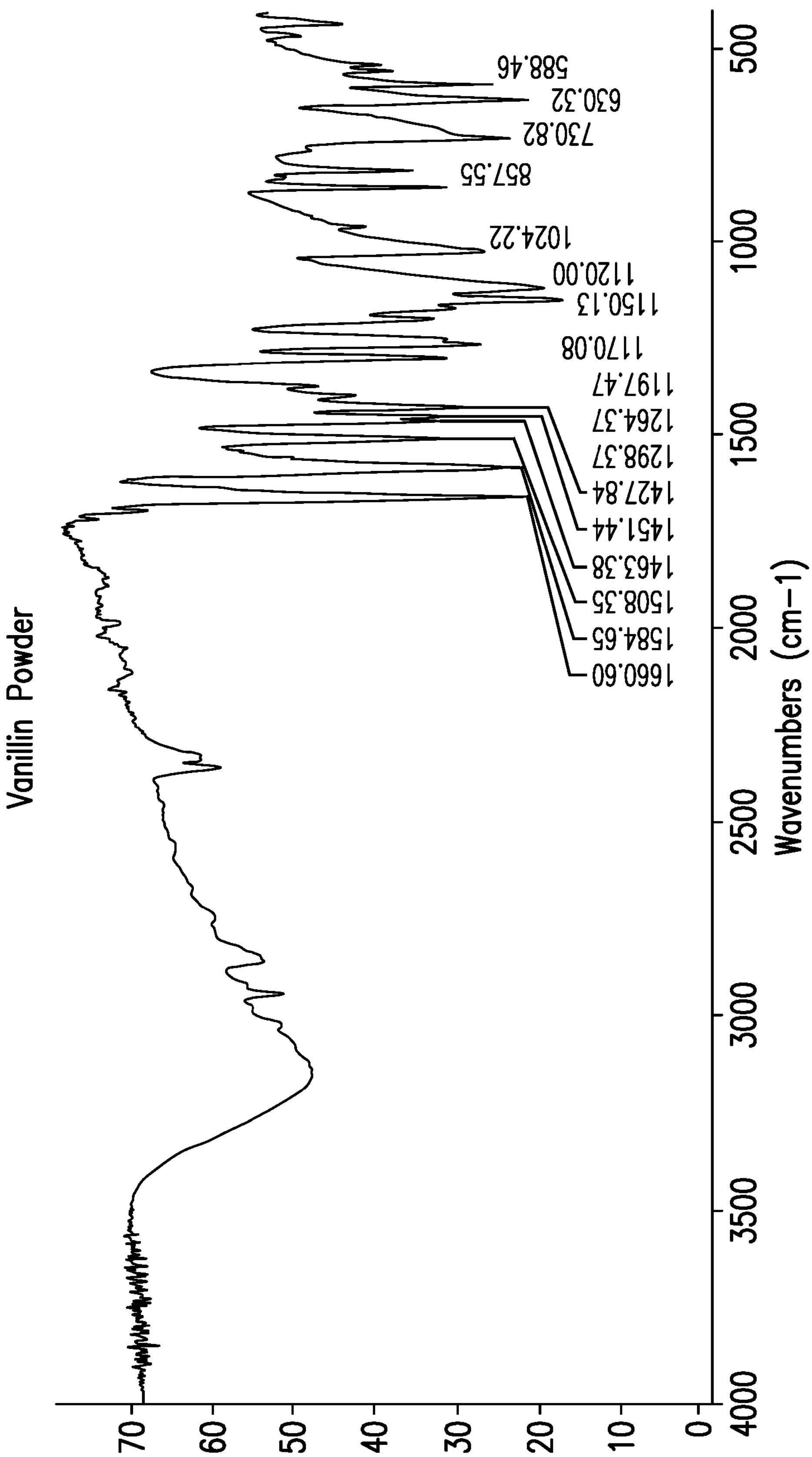
Vanillin Powder
% Reflectance
70
60
50
40
30
20
10
0
4000
3500
3000
2500
2000
1500
1000
500
Wavenumbers (cm−1)
1660.60
1584.65
1508.35
1463.38
1451.44
1427.84
1298.37
1264.37
1197.47
1170.08
1150.13
1120.00
1024.22
857.55
730.82
630.32
588.46

FIG. 7

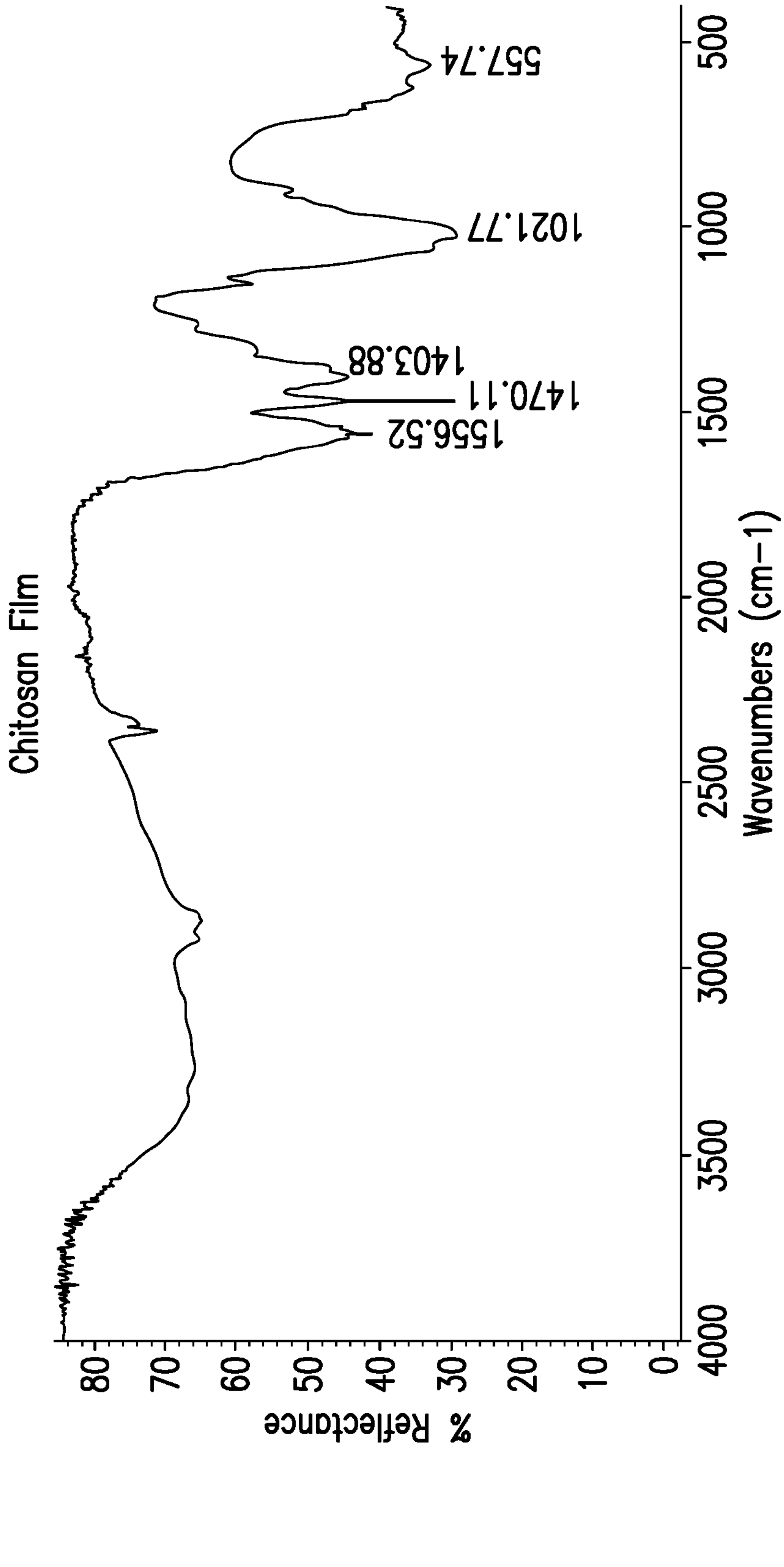
Chitosan Film
% Reflectance
80
70
60
50
40
30
20
10
0
4000
3500
3000
2500
2000
1500
1000
500
Wavenumbers (cm−1)
1556.52
1470.11
1403.88
1021.77
557.74

FIG.8

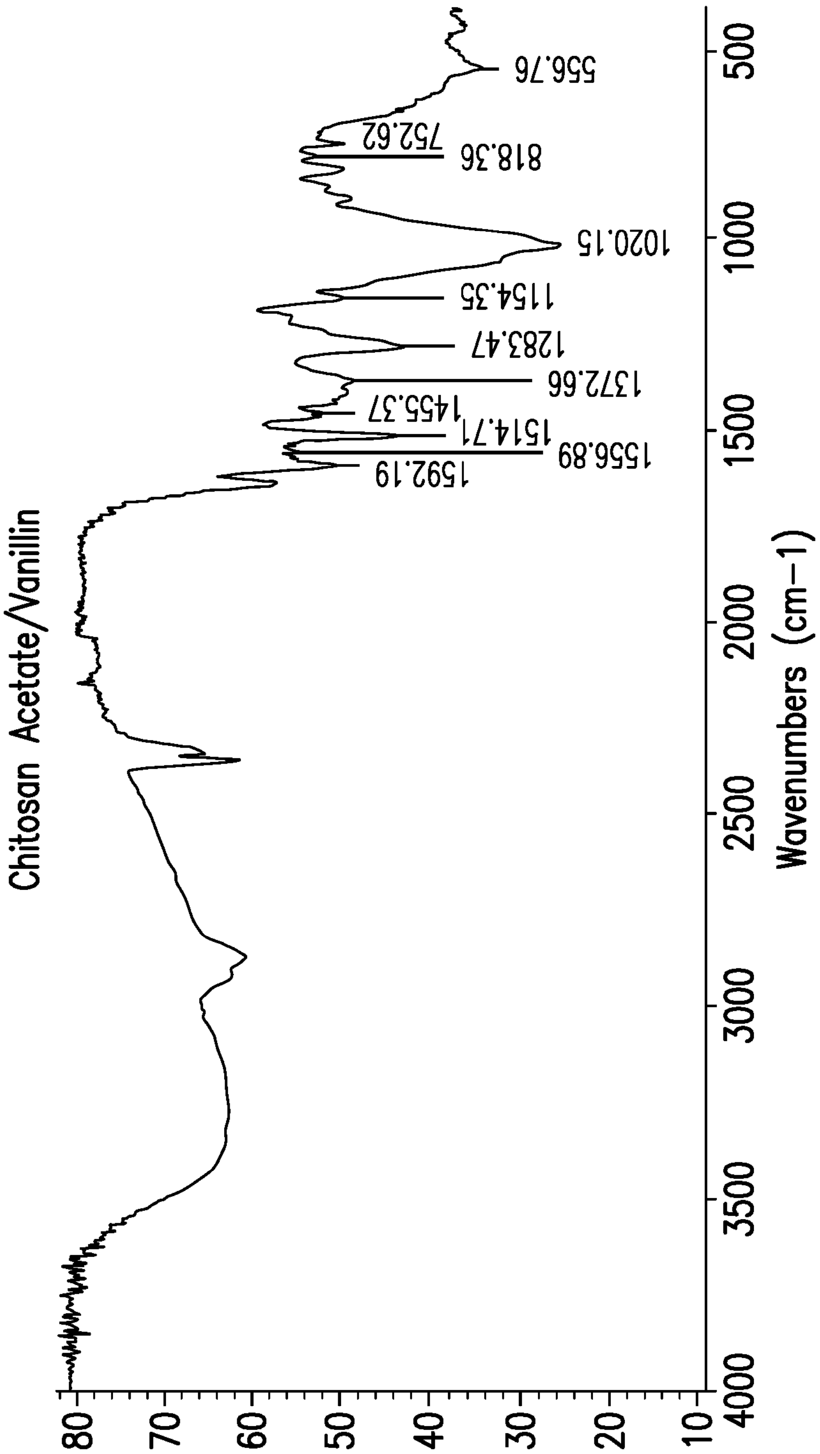
Chitosan Acetate/Vanillin
% Reflectance
80
70
60
50
40
30
20
10
Wavenumbers (cm−1)
4000
3500
3000
2500
2000
1500
1000
500
1592.19
1556.89
1514.71
1455.37
1372.66
1283.47
1154.35
1020.15
818.36
752.62
556.76

FIG.9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013165424 A1
- DE 10259199 A1


### Non-patent literature cited in the description


- **R. J. CRAWFORD** ; **C. R. ROBBINS**. A replacement for Rubine dye for detecting cationics on keratin. *J. Soc. Cosmet, Chem.*, 1980, vol. 31, 273-278
- **N. LECHOCHINSKI** ; **S.BREUGNOT**. *J.Cosmet.Sci*, 2011
- **N. LECHOCHINSKI** ; **S. BREUGNOT**. *J.Cosmet.Sci*, March 2011